(19) Europäisches Patentamt
European Patent Office
Office européen des brevets

(11) Veröffentlichungsnummer: **0 315 839**
**A2**

(12) **EUROPÄISCHE PATENTANMELDUNG**

(21) Anmeldenummer: **88117991.5**

(22) Anmeldetag: **28.10.88**

(51) Int. Cl.[4]: **C07D 405/04 , C07D 409/04 , C07D 411/04 , A01N 43/28 , A01N 43/32**

(30) Priorität: **10.11.87 JP 282211/87**
**30.05.88 JP 130168/88**

(43) Veröffentlichungstag der Anmeldung:
**17.05.89 Patentblatt 89/20**

(84) Benannte Vertragsstaaten:
**BE CH DE FR GB IT LI NL SE**

(71) Anmelder: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Kurahashi, Yoshio**
**47-15, Oya-machi**
**Hachioji-shi Tokyo(JP)**
Erfinder: **Goto, Toshio**
**3454-21, Honmachida**
**Machida-shi Tokyo(JP)**
Erfinder: **Isono, Kunihiro**
**2-32-4, Asahigaoka**
**Hino-shi Tokyo(JP)**
Erfinder: **Kitagawa, Yoshinori**
**1085, Ara-machi**
**Moka-shi Tochigi-ken(JP)**
Erfinder: **Izumi, Tetsuji**
**39-15, Namiki-cho**
**Hachioji-shi Tokyo(JP)**
Erfinder: **Kondo, Toshihito**
**39-15, Namiki-cho**
**Hachioji-shi Tokyo(JP)**
Erfinder: **Sato, Takayo**
**152-2, Shimonojo-machi**
**Takasaki-shi Gunma-ken(JP)**

(74) Vertreter: **Schumacher, Günter, Dr. et al**
**c/o Bayer AG Konzernverwaltung RP**
**Patentabteilung**
**D-5090 Leverkusen 1 Bayerwerk(DE)**

(54) **3-Substituierte Pyridine.**

(57) Offenbart werden neue 3-substituierte Pyridine der Formel (I)

**EP 0 315 839 A2**

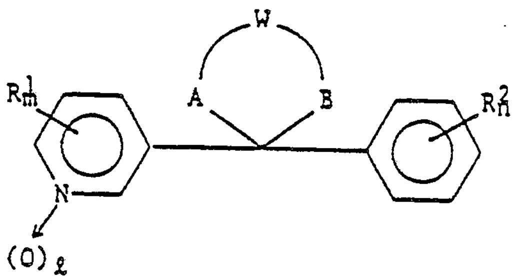

(I)

und die Verwendung der neuen Verbindungen als Fungizide und Herbizide für Landwirtschaft und Gartenbau sowie Verfahren zu ihrer Herstellung.

## 3-Substituierte Pyridine

Die vorliegende Erfindung betrifft neue 3-substituierte Pyridine, mehrere Verfahren zu ihrer Herstellung und ihre Verwendung als Fungizide und Herbizide für Landwirtschaft und Gartenbau.

Es wurde bereits offenbart, daß 2-Phenyl-2-(pyridin-4-yl)-1,3-dioxolan ein Reaktionszwischenprodukt war {siehe Heterocycles 22, 1137 (1984)}, daß 2-Phenyl-2-(pyridin-4-yl)-1,3-dithiolan und 2-Phenyl-2-(pyridin-4-yl)-1,3-dithian Reaktionszwischenprodukte waren {siehe J. Chem. Soc. Perkin Trans. I, 1223 (1984)} und daß 2-Phenyl-2-(pyridin-3-yl)-1,3-dioxolan ein Reaktionszwischenprodukt war {siehe Bull. Soc. Chim. Belg. 89, 67 (1980)}.

Nunmehr wurden neue 3-substituierte Pyridine der Formel (I)

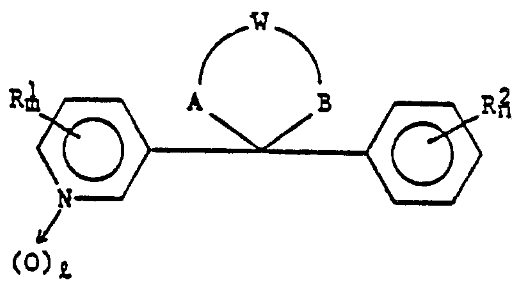

(I)

gefunden, in der A Sauerstoff oder Schwefel bezeichnet,

B Sauerstoff, Schwefel oder Sulfinyl bezeichnet,

$R^1$ Wasserstoff, Halogen, Alkyl oder Halogenoalkyl bezeichnet,

$R^2$ Wasserstoff, Halogen, Alkyl, Phenyl, Halogenoalkyl oder Phenoxy bezeichnet,

$\ell$ 0 oder 1 ist,

m eine ganze Zahl von 1 bis 4 ist,

n eine ganze Zahl von l bis 5 ist und

W eine Gruppe der Formel

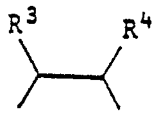

oder

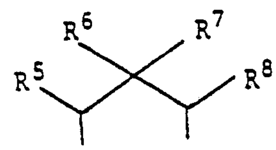

bezeichnet, worin

$R^3$ und $R^4$ jeweils Wasserstoff, Hydroxyalkyl, Alkoxyalkyl, Alkyl, Benzyloxyalkyl, Halogenoalkyl oder Carboxyalkyl bezeichnen oder

$R^3$ und $R^4$ zusammen mit den Kohlenstoff-Atomen, an die sie gebunden sind, einen Kohlenwasserstoff-Ring mit insgesamt 3 bis 12 Kohlenstoff-Atomen bilden und

$R^5$, $R^6$, $R^7$ und $R^8$ jeweils Wasserstoff, Alkyl oder Hydroxyalkyl bezeichnen,

mit der Maßgabe, daß keiner der Substituenten $R^1$, $R^2$, $R^3$ und $R^4$ ein Wasserstoff-Atom bezeichnet, wenn A und B jeweils ein Sauerstoff-Atom bezeichnen und W eine Gruppe der Formel

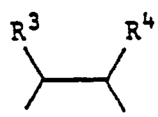

bezeichnet.

3-Substituierte Pyridine der Formel (I) werden dadurch erhalten, daß

a) in dem Fall, in dem $\ell$ 0 ist und B Sauerstoff oder Schwefel bezeichnet,

Verbindungen der Formel (II)

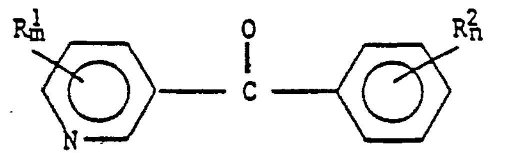

(II)

,

in der $R^1$, $R^2$, m und n die im Vorstehenden angegebenen Bedeutungen haben, mit Verbindungen der Formel (III)

HA - W - B′H (III),

in der A und W die im Vorstehenden angegebenen Bedeutungen haben und
B′ Sauerstoff oder Schwefel bezeichnet, in Gegenwart inerter Lösungsmittel und in Gegenwart von Säure-Katalysatoren umgesetzt werden, oder

b) in dem Fall, in dem ℓ 1 ist und A und B jeweils Sauerstoff bezeichnen,
Verbindungen der Formel (Ib)

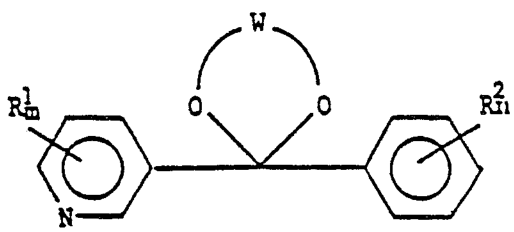

(Ib)

,

in der $R^1$, $R^2$, m, n und W die im Vorstehenden angegebenen Bedeutungen haben, mit einem Oxidationsmittel in Gegenwart inerter Lösungsmittel umgesetzt werden,
oder

c) in dem Fall, in dem B eine Sulfinyl-Gruppe bezeichnet und ℓ 0 ist,
Verbindungen der Formel (Ic)

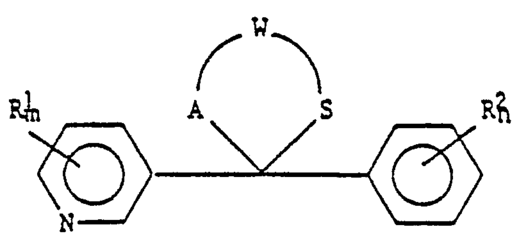

(Ic)

,

in der $R^1$, $R^2$, m, n, A und W die im Vorstehenden angegebenen Bedeutungen haben, mit einem Oxidationsmittel in Gegenwart inerter Lösungsmittel umgesetzt werden.

Die neuen 3-substituierten Pyridine gemäß der Erfindung zeigen potente fungizide und herbizide Eigenschaften.

Überraschenderweise zeigen die erfindungsgemäßen 3-substituierten Pyridine eine wesentlich stärkere fungizide und herbizide Wirkung als die aus dem Stand der Technik bekannten Verbindungen, beispielsweise diejenigen aus den oben genannten Literaturstellen Bull. Soc. Chim. Belg. 89, 67 (1980), J. Chem. Soc. Perkin Trans. I, 1223 (1984) und Heterocycles 22, 1137 (1984).

Unter den erfindungsgemäßen 3-substituierten Pyridinen der Formel (I) sind bevorzugte Verbindungen diejenigen, in denen

A und B jeweils Sauerstoff oder Schwefel bezeichnen,
$R^1$ Wasserstoff oder Chlor bezeichnet,
$R^2$ Wasserstoff, Chlor, Brom, Alkyl mit 1 bis 4 Kohlenstoff-Atomen oder Phenyl bezeichnet,
m und n jeweils 1 oder 2 sind und
W eine Gruppe der Formel

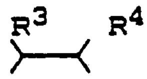

oder

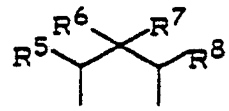

bezeichnet, worin
$R^3$ und $R^4$ jeweils Wasserstoff, Alkyl mit 1 bis 6 Kohlenstoff-Atomen, Hydroxyalkyl mit 1 bis 3 Kohlenstoff-Atomen, Alkoxyalkyl mit insgesamt 2 bis 4 Kohlenstoff-Atomen oder Halogenoalkyl mit 1 bis 4 Kohlenstoff-Atomen bezeichnen oder
$R^3$ und $R^4$ zusammen mit den Kohlenstoff-Atomen, an die sie gebunden sind, einen Kohlenwasserstoff-Ring mit insgesamt 6 bis 12 Kohlenstoff-Atomen bilden und
$R^5$, $R^6$, $R^7$ und $R^8$ jeweils Wasserstoff, Alkyl mit 1 bis 4 Kohlenstoff-Atomen oder Hydroxyalkyl mit 1 bis 4 Kohlenstoff-Atomen bezeichnen,
mit der Maßgabe, daß keiner der Substituenten $R^1$, $R^2$, $R^3$ und $R^4$ Wasserstoff bezeichnet, wenn A und B jeweils Sauerstoff bezeichnen und W eine Gruppe der Formel

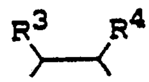

bezeichnet.

Ganz besonders bevorzugte 3-substituierte Pyridine der Formel (I) sind diejenigen, in denen
A und B jeweils Sauerstoff oder Schwefel bezeichnen,
$R^1$ Wasserstoff oder Chlor bezeichnet,
$R^2$ Wasserstoff, Chlor, Brom, Methyl oder Phenyl bezeichnet,
m und n jeweils I oder 2 sind und
W eine Gruppe

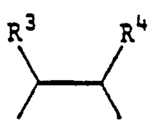

oder

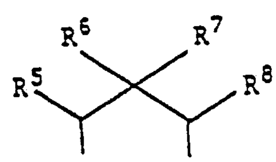

bezeichnet, worin
$R^3$ und $R^4$ jeweils Wasserstoff, Alkyl mit 1 bis 4 Kohlenstoff-Atomen, Methoxymethyl oder Chloromethyl bezeichnen oder
$R^3$ und $R^4$ zusammen mit den Kohlenstoff-Atomen, an die sie gebunden sind, einen Cyclohexan-Ring bilden und
$R^5$, $R^6$, $R^7$ und $R^8$ jeweils Wasserstoff oder Alkyl mit 1 bis 3 Kohlenstoff-Atomen bezeichnen,
mit der Maßgabe, daß keiner der Substituenten $R^1$, $R^2$, $R^3$ und $R^4$ ein Wasserstoff-Atom bezeichnet, wenn A und B jeweils Sauerstoff bezeichnen und W eine Gruppe der Formel

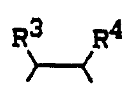

bezeichnet,
Speziell seien die folgenden Verbindungen erwähnt:
2-Phenyl-2-(pyridin-3-yl)-1,3-dithiolan,

4-Methyl-2-phenyl-2-(pyridin-3-yl)-1,3-dithiolan,
2-(2,4-Dichlorphenyl)-5,5-diethyl-2-(3-pyridyl)-1,3-dioxolan,
2-(2-Chlorphenyl)-5,5-dimethyl-2-(3-pyridyl)-1,3-dioxolan,
2-(Biphenyl-4-yl)-5,5-diethyl-2-(3-pyridyl)-1,3-dioxolan und
2-(2,4-Dichlorphenyl)-5-ethyl-5-n-propyl-2-(3-pyridyl)-1,3-dioxolan.

Wenn in dem Verfahren a) beispielsweise 3-Benzoylpyridin und 1,2-Propandiol als Ausgangsstoffe eingesetzt werden, kann der Reaktionsablauf durch die folgende Gleichung dargestellt werden:

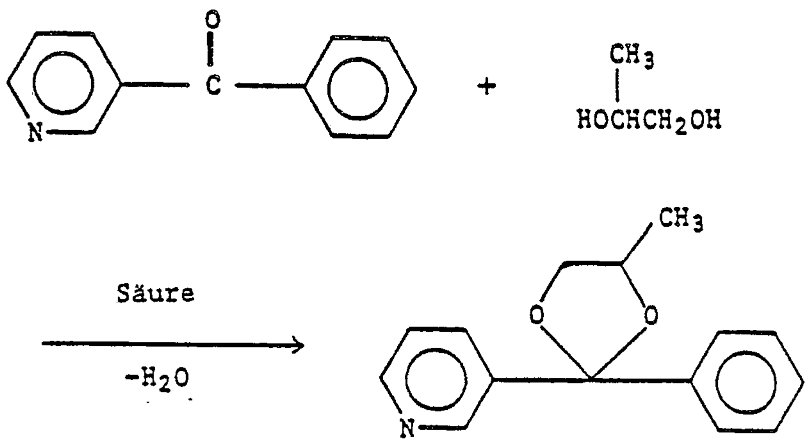


Wenn in dem Verfahren b) beispielsweise 4-tert-Butyl-2-(4-chlorophenyl)-2-(pyridin-3-yl)-1,3-dioxolan und m-Chloroperbenzoesäure als Ausgangsstoffe eingesetzt werden, kann der Reaktionsablauf durch die folgende Gleichung dargestellt werden:

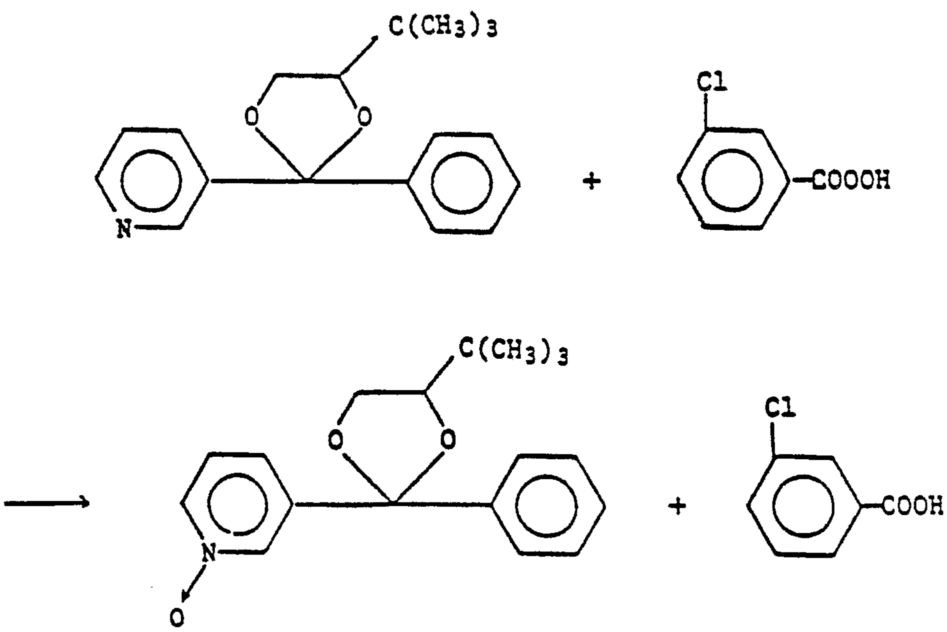

Wenn in dem Verfahren c) beispielsweise 2-Phenyl-2-(pyridin-3-yl)-1,3-dithian und m-Chloroperbenzoesäure als Ausgangsstoffe eingesetzt werden, kann der Reaktionsablauf durch die folgende Gleichung dargestellt werden:

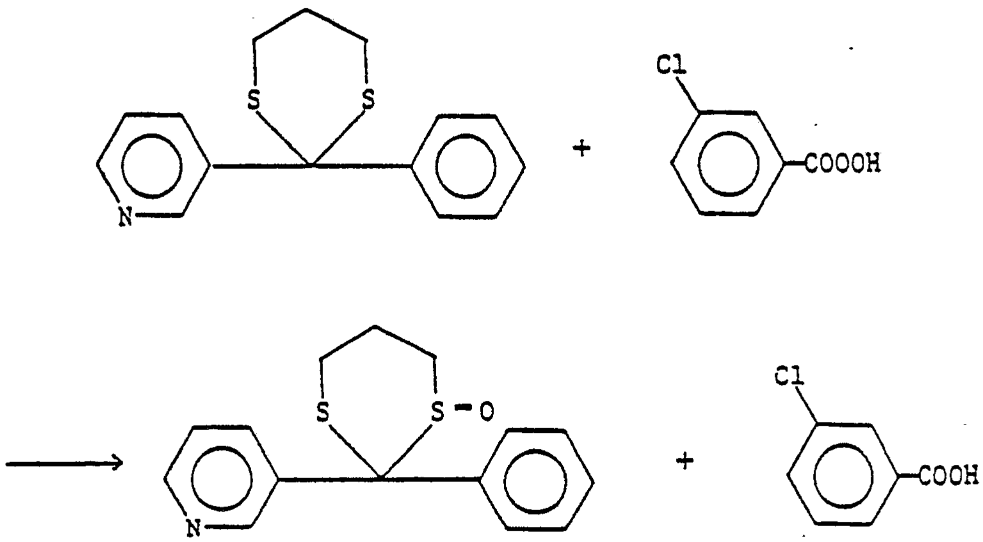

In dem Verfahren a) bezeichnen die Ausgangs-Verbindungen der Formeln (II) und (III) Verbindungen auf der Basis der im Vorstehenden gegebenen Definitionen für $R^1$, $R^2$, m, n, A, $B'$ und W, vorzugsweise Verbindungen auf der Basis der im Vorstehenden gegebenen bevorzugten Definitionen.

Die Verbindungen der Formeln (II) und (III) sind bekannt. 3-Benzoylpyridin mag als Beispiel für die Verbindungen der Formel (II) genannt werden.

1,2-Ethandithiol und 1,2-Propandithiol lassen sich als Beispiele für die Verbindungen der Formel (III) zitieren.

In den Verfahren b) und c) bezeichnen die Ausgangs-Verbindungen der Formeln (Ib) und (Ic) Verbindungen auf der Basis der im Vorstehenden gegebenen Definitionen für $R^1$, $R^2$, m, n, A und W, vorzugsweise Verbindungen auf der Basis der im Vorstehenden gegebenen bevorzugten Definitionen.

Die Verbindungen der Formeln (Ib) und (Ic) können nach dem im Vorstehenden angegebenen Verfahren a) hergestellt werden. 4-tert-Butyl-2-(4-chlorophenyl)-2-(pyridin-3-yl)-1,3-dioxolan kann als Beispiel für eine Verbindung der Formel (Ib) genannt werden, und 2-Phenyl-2-(pyridin-3-yl)-1,3-dithiolan und 4-Methyl-2-phenyl-2-(pyridin-3-yl)-1,3-dithiolan können als Beispiele für eine Verbindung der Formel (Ic) genannt werden.

In den Verfahren b) und c) können Hydrogenperoxid und m-Chloroperbenzoesäure als Oxidationsmittel genannt werden.

Bei der Durchführung des Verfahrens a) ist es moglich, beliebige inerte organische Lösungsmittel als geeignete Verdünnungsmittel zu verwenden. Beispiele für die Verdünnungsmittel sind aliphatische, cycloaliphatische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Chlorbenzol und dergleichen; Ether wie Diethylether, Methylethylether, Di-isopropylether, Dibutylether, Propylenoxid, Dioxan, Tetrahydrofuran und dergleichen; Ketone wie Aceton, Methylethylketon, Methylisopropylketon, Methylisobutylketon und dergleichen; Nitrile wie Acetonitril, Propionitril und Acrylnitril; Alkohole wie Isopropanol; Ester wie Ethylacetat, Amylacetat und dergleichen; Säureamide wie Dimethylformamid und Dimethylacetamid; sowie Sulfone und Sulfoxide wie Dimethylsulfoxid, Sulfolan und dergleichen.

Das Verfahren a) kann in Gegenwart eines Säure-Katalysators durchgefuhrt werden. Als solche Kataylsatoren können beispielsweise Schwefelsäure, Salzsäure, ein Säure-Ionen austauschendes Harz, Methansulfonsäure, Ammoniumchlorid, p-Toluolsulfonsäure, Bortrifluorid-Ether-Komplex, Aluminiumchlorid, Zinkchlorid und Pyridinium-p-toluolsulfonat genannt werden.

Bei dem Verfahren (a) kann die Reaktionstemperatur innerhalb eines Bereichs von beträchtlicher Breite variiert werden. Beispielsweise kann die Reaktion bei einer Temperatur zwischen etwa -10 °C und etwa 200 °C, vorzugsweise bei einer Temperatur von etwa 50 °C bis etwa 150 °C, durchgeführt werden. Vorzugsweise wird die Reaktion unter normalem atmosphärischen Druck durchgeführt, obwohl auch die Anwendung eines höheren oder niedrigeren Druckes zulässig ist.

Bei der Durchführung der Reaktion des Verfahrens (a) werden beispielsweise die Verbindungen der Formel (III) in einer Stoffmenge des 1- bis 10fachen und ein Säure-Katalysator in einer Menge des 1- bis 3fachen der Stoffmenge der oben bezeichneten Verbindungen der Formel (II) in Gegenwart eines inerten Lösungsmittels wie beispielsweise Toluol eingesetzt, um die angestrebte Verbindung zu erhalten.

Bei der Durchführung der Reaktion der oben beschriebenen Verfahren (b) und c) werden als Oxidationsmittel organi sche Peroxide wie m-Chloroperbenzoesäure und Hydrogenperoxid eingesetzt.

Bei der Durchführung der im Vorstehenden erwähnten Verfahren b) und c) unter Verwendung eines organischen Peroxids als Oxidationsmittel können als geeignete Verdünnungsmittel beliebige inerte organische Lösungsmittel verwendet werden.

Wenn das Oxidationsmittel ein organisches Peroxid ist, sind Beispiele für die Verdünnungsmittel aliphatische, cycloaliphatische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Chlorbenzol und dergleichen; Alkohole wie Methanol, Ethanol, Isopropanol, Butanol, Ethylenglycol; Ester wie beispielsweise Ethylacetat, Amylacetat und dergleichen; sowie Säureamide wie Dimethylformamid und Dimethylacetamid.

Bei der Durchführung der oben erwähnten Verfahren b) und c) unter Verwendung von Hydrogenperoxid als Oxidationsmittel können die dabei einzusetzenden Verdünnungsmittel beispielsweise Wasser, das basisch, neutral oder sauer sein kann, Alkohole wie beispielsweise Methanol, Ethanol, Isopropanol, Butanol, Ethylenglycol sowie Carbonsäuren wie beispielsweise Essigsäure sein.

Bei jedem der Verfahren b) und c) kann die Reaktionstemperatur in einem Bereich beträchtlicher Breite variiert werden. Wenn als Oxidationsmittel ein organisches Peroxid eingesetzt wird, kann die Reaktion im allgemeinen bei einer Temperatur zwischen etwa -10 °C und etwa 50 °C, vorzugsweise bei einer Temperatur von etwa 0 °C bis etwa 30 °C, durchgefuhrt werden.

Weiterhin kann, wenn Hydrogenperoxid als Oxidationsmittel angewandt wird, die Reaktion im allgemeinen bei einer Temperatur zwischen etwa -10 °C und etwa 100 °C, vorzugsweise bei einer Temperatur von etwa 0 °C bis etwa 30 °C, durchgeführt werden. Vorzugsweise wird die Reaktion unter normalem Atmosphärendruck durchgefuhrt, jedoch ist auch die Anwendung eines hoheren oder niedrigeren Druckes zulässig.

Bei der Durchführung der Reaktion des Verfahrens b) wird beispielsweise m-Chloroperbenzoesäure in einer Stoffmenge des l bis 2fachen der Stoffmenge der oben bezeichneten Verbindungen der Formel (Ib) in Gegenwart eines inerten Lösungsmittels wie beispielweise Methylenchlorid eingesetzt, um die angestrebte Verbindung zu erhalten.

Bei der Durchführung der Reaktion des oben angegebenen Verfahrens c) wird m-Chloroperbenzoesäure in einer Stoffmenge des 1 bis 1,2fachen der Stoffmenge der oben bezeichneten Verbindungen der Formel (Ic) in Gegenwart eines inerten Lösungsmittels wie beispielweise Methylenchlorid eingesetzt, um die angestrebte Verbindung zu erhalten.

Die aktiven Verbindungen gemäß der Erfindung zeigen eine potente mikrobizide Wirkung und können in der Praxis zur Bekämpfung unerwünschter Mikroorganismen eingesetzt werden.

Fungizide Mittel werden im Pflanzenschutz beispielsweise eingesetzt zur Bekämpfung von
Plasmodiophoromycetes,
Oomycetes,
Chytridiomycetes,
Zygomycetes,
Ascomycetes,
Basidiomycetes und
Deuteromycetes.

Bakterizide Mittel werden im Pflanzenschutz beispielsweise eingesetzt zur Bekämpfung von
Pseudomonadaceae,
Rhizobiaceae,
Enterobacteriaceae,
Corynebacteriaceae und
Streptomycetaceae.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Einige der Organismen, die fungale und bakterielle Erkrankungen verursachen und unter die oben aufgeführten Sammelbegriffe fallen, seien im Folgenden als nichtbeschränkende Beispiele genannt:
Species Xanthomonas wie beispielsweise Xanthomonas campestris pv. oryzae;
Species Pseudomonas wie beispielsweise Pseudomonas syringae pv. lacrymans;
Species Erwinia wie beispielsweise Erwinia amylovora;

Species Pythium wie beispielsweise Pythium ultimum;
Species Phytophthora wie beispielsweise Phytophthora infestans;
Species Pseudoperonospora wie beispielsweise Pseudoperonospora cubensis;
Species Plasmopara wie beispielsweise Plasmopara viticola;
Species Peronospora wie beispielsweise Peronospora pisi oder P. brassicae;
Species Erysiphe wie beispielsweise Erysiphe graminis;
Species Sphaerotheca wie beispielsweise Sphaerotheca fuliginea;
Species Podosphaera wie beispielsweise Podosphaera leucotricha;
Species Venturia wie beispielsweise Venturia inaequalis;
Species Pyrenophora wie beispielsweise Pyrenophora teres oder P. graminea (Conidiale Form: Drechslera, Synonym: Helminthosporium);
Species Cochliobolus wie beispielsweise Cochliobolus sativus (Conidiale Form: Drechslera, Synonym: Helminthosporium);
Species Uromyces wie beispielsweise Uromyces appendiculatus;
Species Puccinia wie beispielsweise Puccinia recondita;
Species Tilletia wie beispielsweise Tilletia caries;
Species Ustilago wie beispielsweise Ustilago nuda oder Ustilago avenae;
Species Pellicularia wie beispielsweise Pellicularia sasakii;
Species Piricularia wie beispielsweise Piricularia oryzae;
Species Fusarium wie beispielsweise Fusarium culmorum;
Species Botrytis wie beispielsweise Botrytis cinerea;
Species Septoria wie beispielsweise Septoria nodorum;
Species Leptosphaeria wie beispielsweise Leptosphaeria nodorum;
Species Cercospora wie beispielsweise Cercospora canescens;
Species Alternaria wie beispielsweise Alternaria brassicae;
Species Pseudocercosporella wie beispielsweise Pseudocercosporella herpotrichoides.

Die gute Tolerierung der aktiven Verbindungen durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Die aktiven Verbindungen gemäß der Erfindung können auch als Unkraut-Vernichtungsmittel, beispielsweise in Verbindung mit den folgenden Pflanzen,verwendet werden:

Dikotyledonen-Unkrauter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obst gärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können umgewandelt werden in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der vorliegenden Erfindung können als Fungizide in ihren oben beschriebenen Formulierungen oder ihren verschiedenen Anwendungsformen als Mischungen gemeinsam mit anderen aktiven Verbindungen vorliegen, etwa mit Fungiziden. Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die aktiven Verbindungen können als solche oder in Form ihrer Präparate oder der daraus durch weitere Verdünnung hergestellten Verwendungsformen eingesetzt werden, beispielsweise als gebrauchsfertige Lösungen, Emulsionen, Suspensionen, Pulver, Pasten und Granulat. Sie gelangen in üblicher Weise zur

Anwendung, beispielsweise durch Bewässern, Eintauchen, Spritzen, Zerstäuben, Vernebeln, Verdampfen, Einspritzen, Bilden einer Aufschlämmung, Aufpinseln, Stäuben, Streuen, Trockendüngung, Feuchtdüngung, Naßdüngung, Schlammdüngung oder Überdecken.

Beim Einsatz als Fungizid zur Behandlung von Pflanzenteilen können die Konzentrationen in den Anwendungsformen innerhalb eines Bereichs beträchtlicher Breite variiert werden. Sie betragen im allgemeinen 1 bis 0,0001 Gew.-%, vorzugsweise von 0,5 bis 0,001 Gew.-%.

Für die Saatgut-Behandlung werden im allgemeinen Mengen der aktiven Verbindung von 0,001 bis etwa 50 g, insbesondere 0,01 bis 10 g, auf 1 kg des Saatguts zur Anwendung gebracht.

Für die Boden-Behandlung werden im allgemeinen Konzentrationen der aktiven Verbindung am Ort der Einwirkung von 0,00001 bis 0,1 Gew.-%, insbesondere 0,0001 bis 0,02 Gew.-%, zur Anwendung gebracht.

Die aktiven Verbindungen gemäß der vorliegenden Erfindung können als Herbizide als solche oder in Form ihrer Präparate zur Unkrautbekämpfung auch als Mischungen mit bekannten Herbiziden eingesetzt werden, wobei Fertigpräparate und Tankmischungen möglich sind.

Mischungen mit anderen bekannten aktiven Verbindungen, etwa mit Herbiziden, Fungiziden, Insektiziden, Akariziden, Nematiziden, vogelabweisenden Mitteln, Pflanzennährstoffen und Mitteln zur Verbesserung der Bodenstruktur sind ebenfalls möglich.

Die aktiven Verbindungen können als solche oder in Form ihrer Präparate oder der daraus durch weitere Verdünnung hergestellten Verwendungsformen eingesetzt werden, beispielsweise als gebrauchsfertige Lösungen, Suspensionen, Emulsionen, Pulver, Pasten und Granulat. Sie gelangen in üblicher Weise zur Anwendung, beispielsweise durch Bewässern, Spritzen, Zerstäuben oder Streuen.

Die erfindungsgemäßen aktiven Verbindungen können als Herbizide vor oder nach dem Auflaufen der Pflanzen eingesetzt werden.

Sie können auch vor der Einsaat in den Boden eingearbeitet werden. Sie werden insbesondere nach dem Auflaufen der Pflanzen eingesetzt.

Die Menge der aktiven Verbindung kann innerhalb eines Bereichs von beträchtlicher Breite variiert werden. Sie hängt im wesentlichen von der Natur der gewünschten Wirkung ab. Im allgemeinen liegen die Aufwandsmengen der aktiven Verbindung zwischen 0,1 und 10 kg/ha, vorzugsweise zwischen 0,5 und 5 kg/ha.

Die Herstellung und die Verwendung der erfindungsgemäßen aktiven Verbindungen sind den folgenden Beispielen zu entnehmen.

Herstellungsbeispiele

Beispiel 1

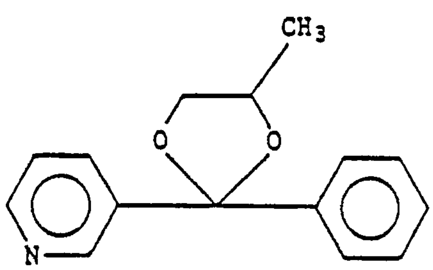

(Verbindung Nr. 6)

1,83 g 3-Benzoylpyridin, 1,44 g 1,2-Propandiol und 2,43 g p-Toluolsulfonsäure-monohydrat wurden in einer Mischung aus 15 ml Toluol und 15 ml n-Butanol gelöst, und die resultierende Mischung wurde zum Rückfluß erhitzt, wobei das dabei gebildete Wasser aus ihr entfernt wurde. Nach Beendigung der Reaktion wurden die Lösungsmittel im Vakuum abdestilliert. Der Rückstand wurde in Dichloromethan gelöst, mit einer wäßrigen Lösung von Natriumhydroxid und dann mit Wasser gewaschen und danach uber wasserfreiem Natriumsulfat getrocknet. Nach dem Abfiltrieren des Natriumsulfats wurde das Lösungsmittel im Vakuum abdestilliert, wonach 4-Methyl-2-phenyl-(2-pyridin-3-yl)-1,3-dioxolan erhalten wurde; Schmp. 40-44 °C.

Beispiel 2

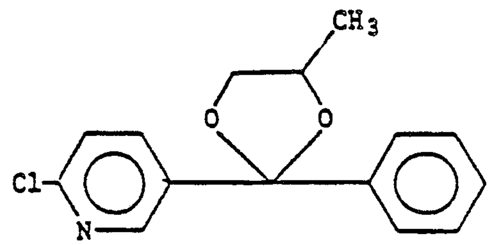

(Verbindung Nr. 13)

Zu 25 ml einer Lösung von 1,5 g 3-Benzoyl-6-chloropyridin und 3,2 g 1,2-Propandiol in Toluol wurden 1,2 g Bortrifluorid-Etherat hinzugefügt. Die resultierende Mischung wurde 6 h zum Rückfluß erhitzt, wobei das dabei gebildete Wasser aus ihr entfernt wurde. Dann wurde die Reaktionsmischung gekühlt, in eine wäßrige Lösung von Natriumhydroxid gegossen und mit Ether extrahiert. Die organische Phase wurde mit Wasser gewaschen und über wasserfreiem Natriumsulfat getrocknet. Nach dem Abfiltrieren des Natriumsulfats und dem Abdestillieren des Lösungsmittels im Vakuum wurde 2-(6-Chloropyridin-3-yl)-4-methyl-2-phenyl-1,3-dioxolan erhalten; Schmp. 44-51 °C.

Beispiel 3

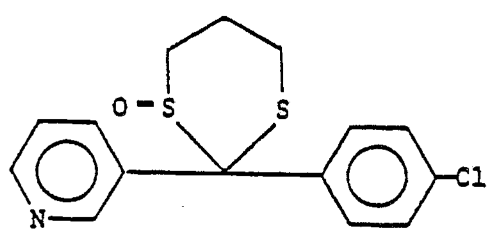

(Verbindung Nr. 5)

Zu 10 ml einer Lösung von 1,1 g 2-(4-Chlorophenyl)-2-(pyridin-3-yl)-1,3-dithian in Methylenchlorid wurden 1,3 g m-Chloroperbenzoesäure unter Eiskühlung hinzugefügt. Die resultierende Mischung wurde 30 min unter Eiskühlung und dann l h bei Raumtemperatur gerührt und danach zum Rückfluß erhitzt. Nach Beendigung der Reaktion wurden die dabei abgeschiedenen Kristalle abfiltriert, wonach 2-(4-Chlorophenyl)-2-(pyridin-3-yl)-1,3-dithian-1-oxid erhalten wurde; $n_D^{20}$ : 1,3305.

Beispiel 4

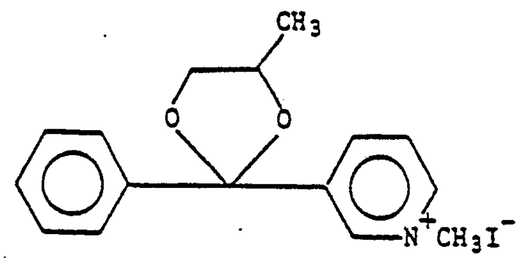

(Verbindung Nr. 7)

2,4 g 4-Methyl-2-phenyl-2-(pyridin-3-yl)-1,3-dioxolan wurden in einer kleinen Menge Ethanol gelöst, und 1,7 g Methyliodid wurde hinzugefügt. Die resultierende Mischung wurde 4 h bei Raumtemperatur gerührt. Nach dem Abdestillieren des Lösungsmittels wurden die dabei erhaltenen Kristalle mit trockenem Ethanol gewaschen, wonach 4-Methyl-2-phenyl-2-(pyridin-3-yl)-1,3-dioxolanmethyliodid erhalten wurde; Schmp. 125-132 °C.

Beispiel 5

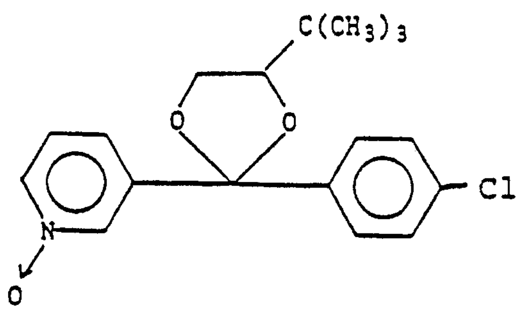

(Verbindung Nr. 20)

Zu 10 ml einer Lösung von 0,63 g 4-tert-Butyl-2-(4-chlorophenyl)-2-(pyridin-3-yl)-1,3-dioxolan in Methylenchlorid wurden 0,22 g m-Chloroperbenzoesäure hinzugefügt, und die resultierende Mischung wurde bei Raumtemperatur gerührt. Nach Beendigung der Reaktion wurde die Reaktionsmischung in eine wäßrige Lösung von Natriumhydroxid gegossen und mit Methylenchlorid extrahiert. Die organische Phase wurde mit Wasser gewaschen und über wasserfreiem Natriumsulfat getrocknet. Nach dem Abfiltrieren des Natriumsulfats und dem Abdestillieren des Lösungsmittels im Vakuum wurde 4-tert-Butyl-2-(4-chlorophenyl)-2-(pyridin-N-oxid-3-yl)-1,3-dioxolan erhalten.

Tabelle 1 zeigt die Verbindungen der vorliegenden Erfindung, die nach den gleichen Methoden wie denjenigen der vorstehenden Beispiele erhalten werden.

Tabelle 1

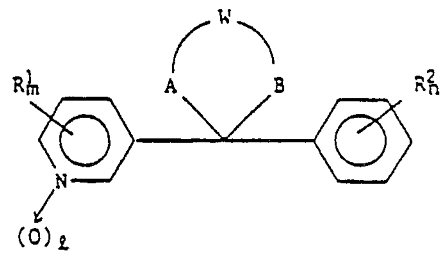

| Verb. Nr. | 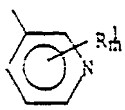 | 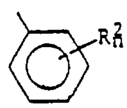 | A | B | W | Aussehen |
|---|---|---|---|---|---|---|
| 1 | 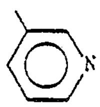 | 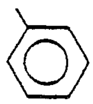 | S | S | $-CH_2CH_2-$ | Schmp. 60 - 61 |
| 2 | 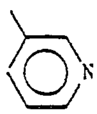 | 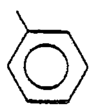 | S | S | $-CH_2\overset{\overset{CH_3}{\mid}}{C}H-$ | $n_D^{20}$ 1.6439 |
| 3 | 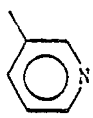 | 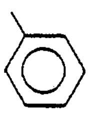 | S | S | $-CH_2CH_2CH_2-$ | Schmp. 96 - 98 |
| 4 | 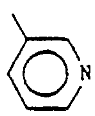 | 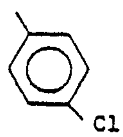 | S | S | $-CH_2CH_2CH_2-$ | $n_D^{20}$ 1.5820 |
| 5 | 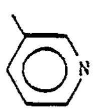 | 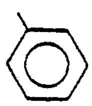 | S | $-\overset{\overset{O}{\mid}}{S}-$ | $-CH_2CH_2CH_2-$ | |

Tabelle 1 (Fortsetzung)

| | | | | | | |
|---|---|---|---|---|---|---|
| 6 | 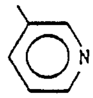 | 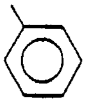 | 0 | 0 | $CH_3$<br>$-CH_2CH-$ | Schmp. 40 - 44 |
| 7 | 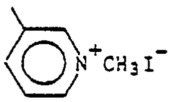 | 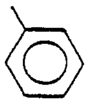 | 0 | 0 | $CH_3$<br>$-CH_2CH-$ | Schmp. 125 - 132 |
| 8 | 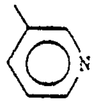 | 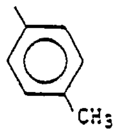 | 0 | 0 | $CH_3$<br>$-CH_2CH-$ | $n_D^{20}$ 1.5600 |
| 9 | 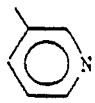 | 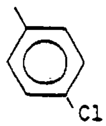 | 0 | 0 | $CH_3$<br>$-CH_2CH-$ | $n_D^{20}$ 1.5675 |
| 10 | 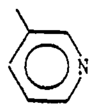 | 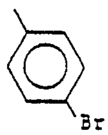 | 0 | 0 | $CH_3$<br>$-CH_2CH-$ | $n_D^{20}$ 1.5812 |
| 11 | 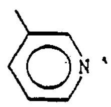 | 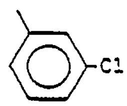 | 0 | 0 | $CH_3$<br>$-CH_2CH-$ | $n_D^{20}$ 1.5680 |
| 12 | 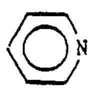 | 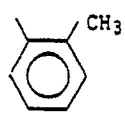 | 0 | 0 | $CH_3$<br>$-CH_2CH-$ | $n_D^{20}$ 1.5646 |
| 13 | 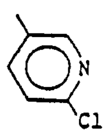 | 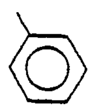 | 0 | 0 | $CH_3$<br>$-CH_2CH-$ | Schmp. 44 - 51 |

Tabelle 1 (Fortsetzung)

| | | | | | | |
|---|---|---|---|---|---|---|
| 14 | 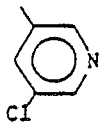 | 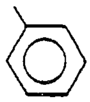 | O | O | $CH_3$<br>$-CH_2CH-$ | $n_D^{20}$ 1.5730 |
| 15 | 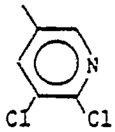 | 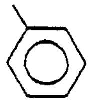 | O | O | $CH_3$<br>$-CH_2CH-$ | $n_D^{20}$ 1.5718 |
| 16 | 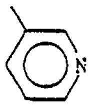 | 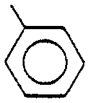 | O | O | $CH_2CH_3$<br>$-CH_2CH-$ | $n_D^{20}$ 1.5485 |
| 17 | 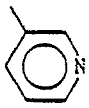 | 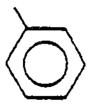 | O | O | $CH_2CH_2CH_3$<br>$-CH_2CH-$ | $n_D^{20}$ 1.5290 |
| 18 | 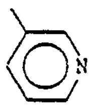 | 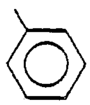 | O | O | $CH_2CH_2CH_2CH_3$<br>$-CH_2CH-$ | $n_D^{20}$ 1.5305 |
| 19 | 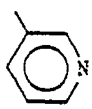 | 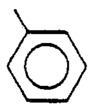 | O | O | $C(CH_3)_3$<br>$-CH_2CH-$ | Schmp. 42 - 47 |
| 20 | 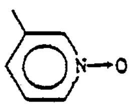 | 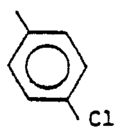 | O | O | $C(CH_3)_3$<br>$-CH_2CH-$ | $n_D^{20}$ 1.545<br>ölig |
| 21 | 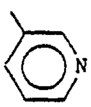 | 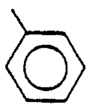 | O | O | $CH_2(CH_2)_4CH_3$<br>$-CH_2CH-$ | $n_D^{20}$ 1.5188 |

Tabelle 1 (Fortsetzung)

| | | | | | | |
|---|---|---|---|---|---|---|
| 22 | 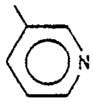 | 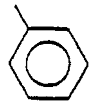 | O | O | 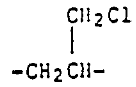 | $n_D^{20}$ 1.5705 |
| 23 | 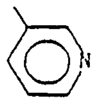 | 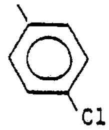 | O | O | 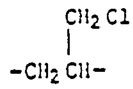 | ölig |
| 24 | 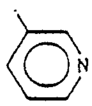 | 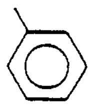 | O | O | 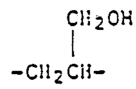 | $n_D^{20}$ 1.5757 |
| 25 | 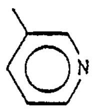 | 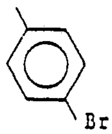 | O | O | 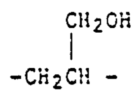 | $n_D^{20}$ 1.5931 |
| 26 | 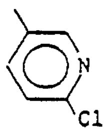 | 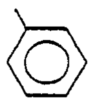 | O | O | 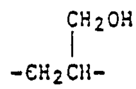 | $n_D^{20}$ 1.5820 |
| 27 | 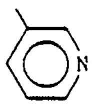 | 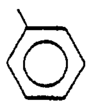 | O | O | 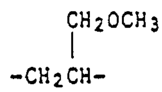 | $n_D^{20}$ 1.5550 |
| 28 | 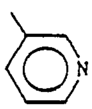 | 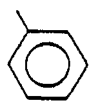 | O | O | 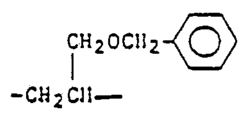 | $n_D^{20}$ 1.5784 |
| 29 | 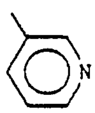 | 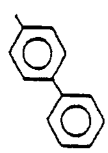 | S | 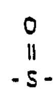 | 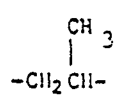 | |

Tabelle 1 (Fortsetzung)

| | | | | | | |
|---|---|---|---|---|---|---|
| 30 | 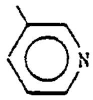 | 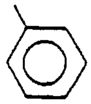 | 0 | 0 | 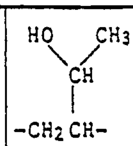 | $n_D^{20}$ 1.5578 |
| 31 | 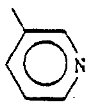 | 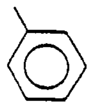 | 0 | 0 | 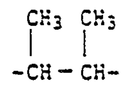 | Schmp. 108 - 115 |
| 32 | 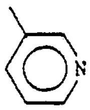 | 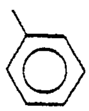 | 0 | 0 | 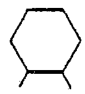 | Schmp. 111 - 119 |
| 33 | 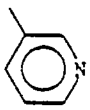 | 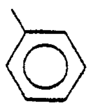 | 0 | 0 | 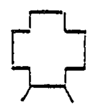 | $n_D^{50}$ 1.525 - 1.530 |
| 34 | 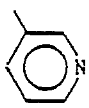 | 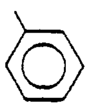 | 0 | 0 | $-CH_2CH_2CH_2-$ | Schmp. 73 - 81 |
| 35 | 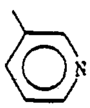 | 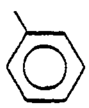 | 0 | 0 | 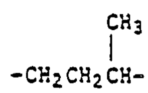 | Schmp. 67 - 73 |
| 36 | 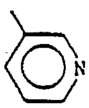 | 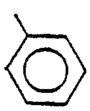 | 0 | 0 | 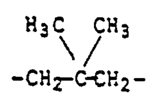 | Schmp. 88 - 92 |

Tabelle 1 (Fortsetzung)

| | | | | | | |
|---|---|---|---|---|---|---|
| 37 | 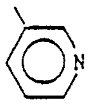 | 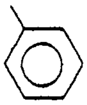 | O | O | 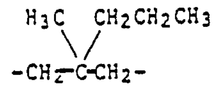 | $n_D^{50}$ 1.5350 |
| 38 | 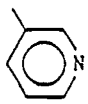 | 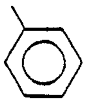 | O | O | 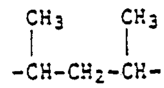 | Schmp. 102 - 104 |
| 39 | 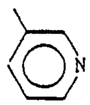 | 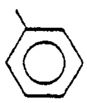 | O | O | 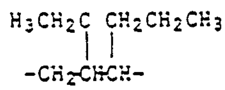 | $n_D^{20}$ 1.5403 |
| 40 | 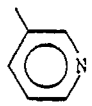 | 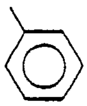 | O | O | 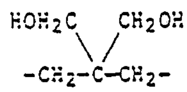 | $n_D^{20}$ 1.5765 |
| 41 | 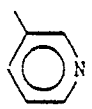 | 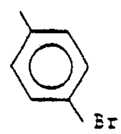 | O | O | 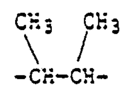 | $n_D^{20}$ 1.5563 |
| 42 | 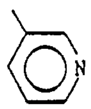 | 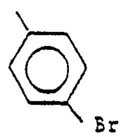 | O | O | 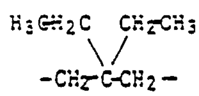 | $n_D^{20}$ 1.5340 |
| 43 | 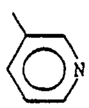 | 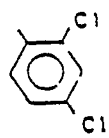 | S | 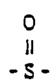 | 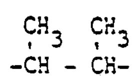 | |
| 44 | 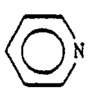 | 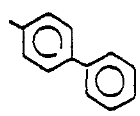 | S | 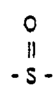 | 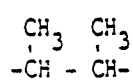 | |

Tabelle 1 (Fortsetzung)

| | | | | | | |
|---|---|---|---|---|---|---|
| 45 | 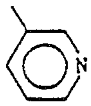 | 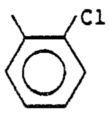 | 0 | 0 | $C(CH_3)_3$<br>$-CH_2CH-$ | |
| 46 | 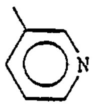 | 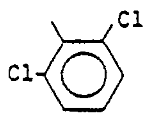 | 0 | 0 | $C(CH_3)_3$<br>$-CH_2CH-$ | |
| 47 | 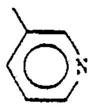 | 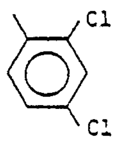 | 0 | 0 | $C(CH_3)_3$<br>$-CH_2CH-$ | $n_D^{20}$ 1.5455 |
| 48 | 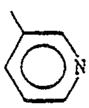 | 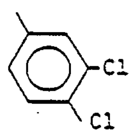 | 0 | 0 | $C(CH_3)_3$<br>$-CH_2CH-$ | $n_D^{20}$ 1.5330 |
| 49 | 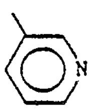 | 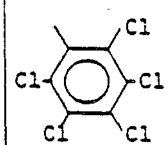 | 0 | 0 | $C(CH_3)_3$<br>$-CH_2CH-$ | |
| 50 | 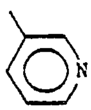 | 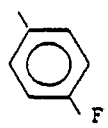 | 0 | 0 | $C(CH_3)_3$<br>$-CH_2CH-$ | |
| 51 | 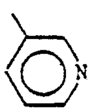 | 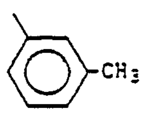 | 0 | 0 | $C(CH_3)_3$<br>$-CH_2CH-$ | |
| 52 | 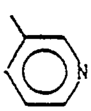 | 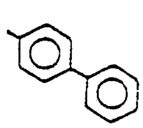 | 0 | 0 | $C(CH_3)_3$<br>$-CH_2CH-$ | Schmp. 87 - 100 |

Tabelle 1 (Fortsetzung)

| | | | | | | |
|---|---|---|---|---|---|---|
| 53 | 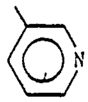 | 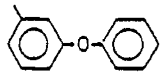 | O | O | $C(CH_3)_3$<br>\|<br>$-CH_2CH-$ | $n_D^{20}$ 1.5592 |
| 54 | 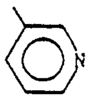 | 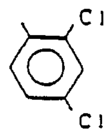 | S | O<br>‖<br>-S- | $-CH_2-CH_2-CH_2-$ | |
| 55 | 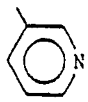 | 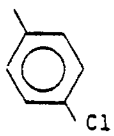 | O | O | $C(CH_3)_3$<br>\|<br>$-CH_2CH-$ | $n_D^{20}$ 1.5750 |
| 56 | 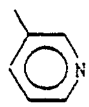 | 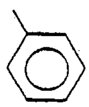 | O | O | $CH_3CH_2$ $CH_2CH_3$<br>\ /<br>$-CH_2-C-CH_2-$ | $n_D^{20}$ 1.5519 |
| 57 | 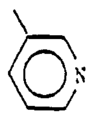 | 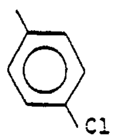 | S | S | $-CH_2-CH_2-$ | $n_D^{20}$ 1.6545 |
| 58 | 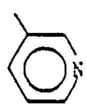 | 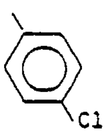 | S | S | $CH_3$<br>\|<br>$-CH_2-CH-$ | $n_D^{22.5}$ 1.6394 |
| 59 | 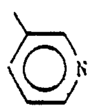 | 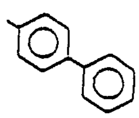 | S | S | $CH_3$<br>\|<br>$-CH_2-CH-$ | $n_D^{20}$ 1.6752 |
| 60 | 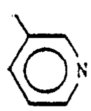 | 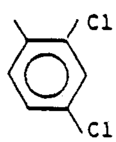 | S | S | $CH_3$<br>\|<br>$-CH_2-CH-$ | $n_D^{20}$ 1.6256 |

Tabelle 1 (Fortsetzung)

| | | | | | | |
|---|---|---|---|---|---|---|
| 61 | 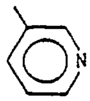 | 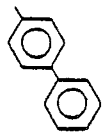 | S | $-\overset{O}{\overset{\|}{S}}-$ | $-CH_2CH_2CH_2-$ | |
| 62 | 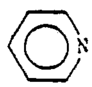 | 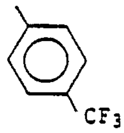 | S | S | $-CH_2CH_2-$ | |
| 63 | 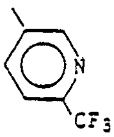 | 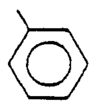 | S | S | $-CH_2CH_2-$ | |
| 64 | 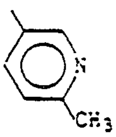 | 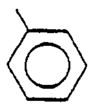 | S | S | $-CH_2CH_2-$ | |
| 65 | 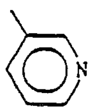 | 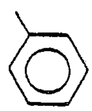 | S | $-\overset{O}{\overset{\|}{S}}-$ | $-CH_2CH_2-$ | |
| 66 | 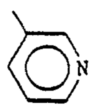 | 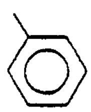 | S | $-\overset{O}{\overset{\|}{S}}-$ | $-CH_2\overset{CH_3}{\overset{\|}{C}H}-$ | |
| 67 | 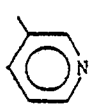 | 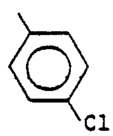 | S | $-\overset{O}{\overset{\|}{S}}-$ | $-CH_2CH_2CH_2-$ | $n_D^{20}$ 1.3305 |
| 68 | 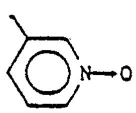 | 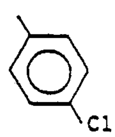 | O | O | $-CH_2\overset{CH_2Cl}{\overset{\|}{C}H}-$ | |

Tabelle 1 (Fortsetzung)

| | | | | | | |
|---|---|---|---|---|---|---|
| 69 | 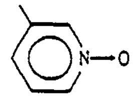 | 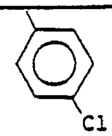 | O | O | 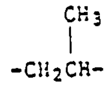 | |
| 70 | 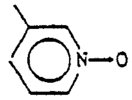 | 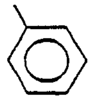 | O | O | 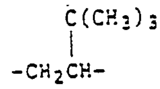 | |
| 71 | 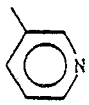 | 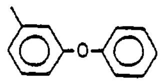 | S | S | 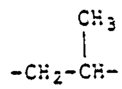 | $n_D^{20}$ 1.5828 |
| 72 | 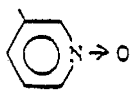 | 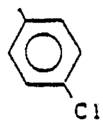 | O | O | 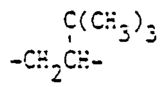 | ölig |
| 73 | 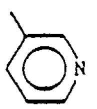 | 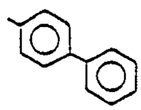 | O | O | 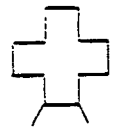 | Schmp. 81 - 90°C |
| 74 | 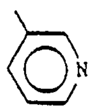 | 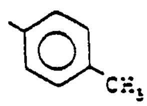 | O | O | 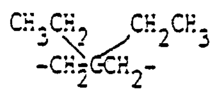 | $n_D^{20}$ 1,5470 |
| 75 | 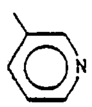 | 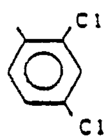 | O | O | 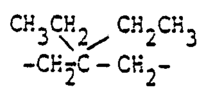 | $n_D^{20}$ 1,5720 |
| 76 | 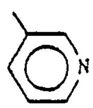 | 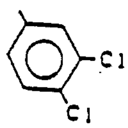 | O | O | 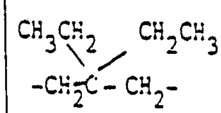 | $n_D^{20}$ 1,5548 |

Tabelle 1 (Fortsetzung)

| | | | | | | |
|---|---|---|---|---|---|---|
| 77 | 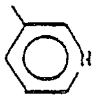 | 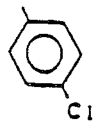 | O | O | $-CH_2CH_2-$ | |
| 78 | 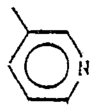 | 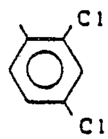 | O | O | $-CH_2CH_2-$ | |
| 79 | 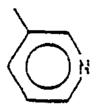 | 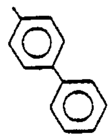 | O | O | $-CH_2CH_2-$ | |
| 80 | 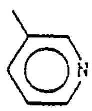 | 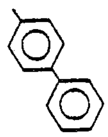 | O | O | $CH_3$<br>\|<br>$-CH_2CH-$ | |
| 81 | 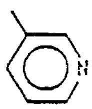 | 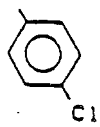 | O | O | $CH_2CH_3$<br>\|<br>$-CH_2CH-$ | |
| 82 | 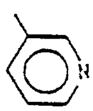 | 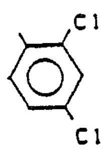 | O | O | $CH_2CH_3$<br>\|<br>$-CH_2CH-$ | |
| 83 | 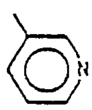 | 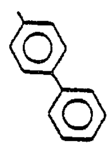 | O | O | $CH_2CH_3$<br>\|<br>$-CH_2CH-$ | |
| 84 | 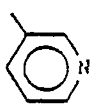 | 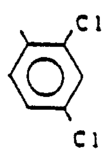 | O | O | $CH_2CH_2CH_3$<br>\|<br>$-CH_2CH-$ | |

Tabelle 1 (Fortsetzung)

| | | | | | | |
|---|---|---|---|---|---|---|
| 85 | 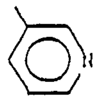 | 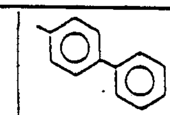 | O | O | $-CH_2CH(CH_2CH_2CH_3)-$ | |
| 86 | 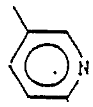 | 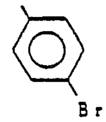 | O | O | $-CH_2CH(CH(CH_3)_2)-$ | |
| 87 | 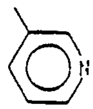 | 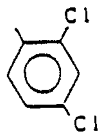 | O | O | $-CH_2CH(CH(CH_3)_2)-$ | |
| 88 | 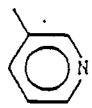 | 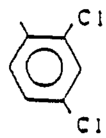 | O | O | $-CH_2CH(CH_2CH_2CH_2CH_3)-$ | |
| 89 | 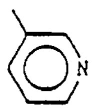 | 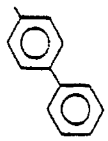 | O | O | $-CH_2CH(CH_2CH_2CH_2CH_3)-$ | |
| 90 | 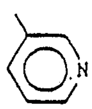 | 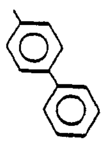 | O | O | $-CH_2CH(CH_2CH(CH_3)_2)-$ | |
| 91 | 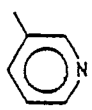 | 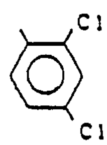 | O | O | $-CH_2CH(CH_3CHCH_2CH_3)-$ | |
| 92 | 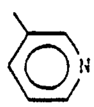 | 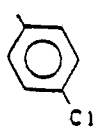 | O | O | $-CH_2CH(CH_2Cl)-$ | |

Tabelle 1 (Fortsetzung)

| | | | | | | |
|---|---|---|---|---|---|---|
| 93 | 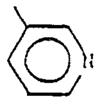 | 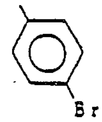 | 0 | 0 | $CH_2Cl$<br>$-CH_2CH-$ | |
| 94 | 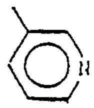 | 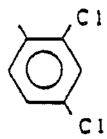 | 0 | 0 | $CH_2Cl$<br>$-CH_2CH-$ | |
| 95 | 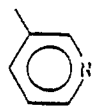 | 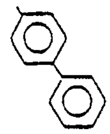 | 0 | 0 | $CH_2Cl$<br>$-CH_2CH-$ | |
| 96 | 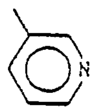 | 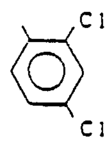 | 0 | 0 | $-CH_2CH_2CH_2-$ | |
| 97 | 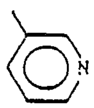 | 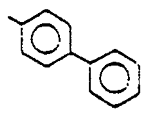 | 0 | 0 | $-CH_2CH_2CH_2-$ | Schmp.143 - 144.5°C |
| 98 | 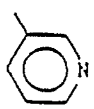 | 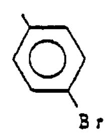 | 0 | 0 | $CH_3$<br>$-CH_2CH_2CH-$ | |
| 99 | 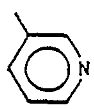 | 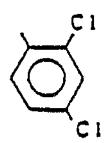 | 0 | 0 | $CH_3$<br>$-CH_2CH_2CH-$ | |
| 100 | 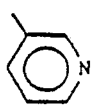 | 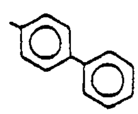 | 0 | 0 | $CH_3$<br>$-CH_2CH_2CH-$ | Schmp.111 - 115°C |

Tabelle 1 (Fortsetzung)

| | | | | | | |
|---|---|---|---|---|---|---|
| 101 | 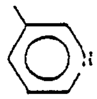 | 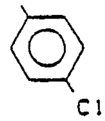 | 0 | 0 | $CH_2CH_3$<br>$-CH_2CH_2CH-$ | |
| 102 | 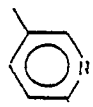 | 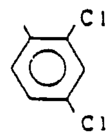 | 0 | 0 | $CH_2CH_3$<br>$-CH_2CH_2CH-$ | |
| 103 | 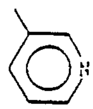 | 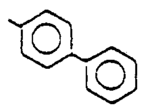 | 0 | 0 | $CH_2CH_3$<br>$-CH_2CH_2CH-$ | |
| 104 | 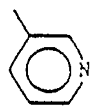 | 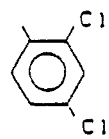 | 0 | 0 | $CH(CH_3)_2$<br>$-CH_2CH_2CH-$ | |
| 105 | 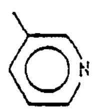 | 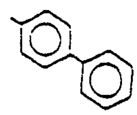 | 0 | 0 | $CH(CH_3)_2$<br>$-CH_2CH_2CH-$ | |
| 106 | 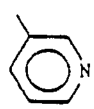 | 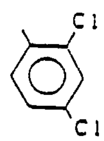 | 0 | 0 | $CH_2CH_2CH_2CH_3$<br>$-CH_2CH_2CH-$ | |
| 107 | 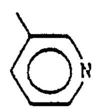 | 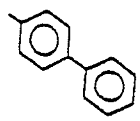 | 0 | 0 | $CH_2CH_2CH_2CH_3$<br>$-CH_2CH_2CH-$ | |
| 108 | 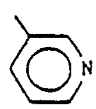 | 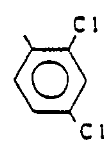 | 0 | 0 | $CH_2CH(CH_3)_2$<br>$-CH_2CH_2CH-$ | |

Tabelle 1 (Fortsetzung)

| | | | | | | |
|---|---|---|---|---|---|---|
| 109 | 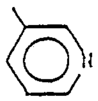 | 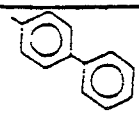 | 0 | 0 | $CH_3CHCH_2CH_3$<br>$-CH_2CH_2CH-$ | |
| 110 | 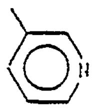 | 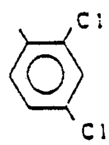 | 0 | 0 | $C(CH_3)_3$<br>$-CH_2CH_2CH-$ | |
| 111 | 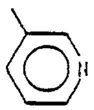 | 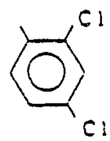 | 0 | 0 | $CH_3$<br>$-CH_2CHCH_2-$ | |
| 112 | 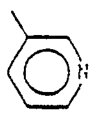 | 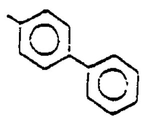 | 0 | 0 | $CH_3$<br>$-CH_2CHCH_2-$ | |
| 113 | 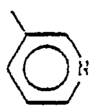 | 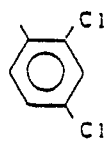 | 0 | 0 | $CH_2CH_3$<br>$-CH_2CHCH_2-$ | |
| 114 | 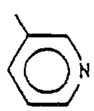 | 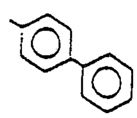 | 0 | 0 | $CH_2CH_3$<br>$-CH_2CHCH_2-$ | |
| 115 | 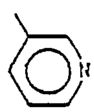 | 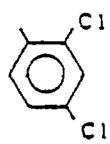 | 0 | 0 | $CH_2CH_2CH_3$<br>$-CH_2CHCH_2-$ | |
| 116 | 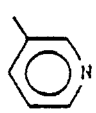 | 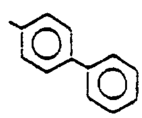 | 0 | 0 | $CH_2CH_2CH_3$<br>$-CH_2CHCH_2-$ | |

Tabelle 1 (Fortsetzung)

| | | | | | | |
|---|---|---|---|---|---|---|
| 125 | 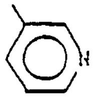 | 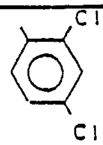 | 0 | 0 | $CH_3CH_3$<br>$-CH_2-CH-CH-$ | |
| 126 | 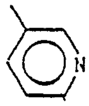 | 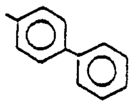 | 0 | 0 | $CH_3$ $CH_3$<br>$-CH_2-CH - CH-$ | |
| 127 | 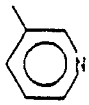 | 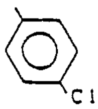 | 0 | 0 | $CH_3CH_2$ $CH_3$<br>$-CH_2-CH- CH-$ | |
| 128 | 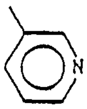 | 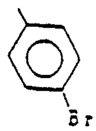 | 0 | 0 | $CH_3CH_2$ $CH_3$<br>$-CH_2-CH - CH-$ | |
| 129 | 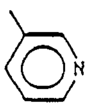 | 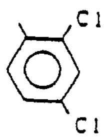 | 0 | 0 | $CH_3CH_2$ $CH_3$<br>$-CH_2-CH - CH-$ | |
| 130 | 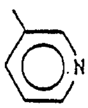 | 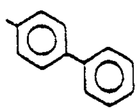 | 0 | 0 | $CH_3CH_2$ $CH_3$<br>$-CH_2-CH - CH-$ | ölig |
| 131 | 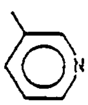 | 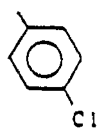 | 0 | 0 | $CH_3CH_2CH_2$ $CH_3$<br>$-CH_2-CH - CH-$ | |
| 132 | 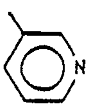 | 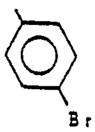 | 0 | 0 | $CH_3CH_2CH_2$ $CH_3$<br>$-CH_2-CH - CH-$ | |

Tabelle 1 (Fortsetzung)

| | | | | | | |
|---|---|---|---|---|---|---|
| 117 | 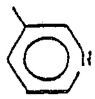 | 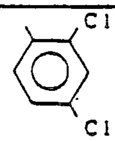 | O | O | $CH(CH_3)_2$<br>$-CH_2-CH-CH_2-$ | |
| 118 | 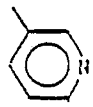 | 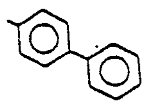 | O | O | $CH(CH_3)_2$<br>$-CH_2-CH-CH_2-$ | Schmp.132 - 140°C |
| 119 | 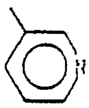 | 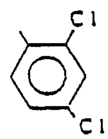 | O | O | $CH_2CH_2CH_2CH_3$<br>$-CH_2-CH-CH_2-$ | |
| 120 | 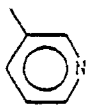 | 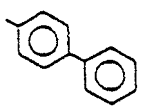 | O | O | $CH_2CH_2CH_2CH_3$<br>$-CH_2-CH-CH_2-$ | |
| 121 | 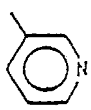 | 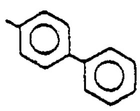 | O | O | $CH_2CH(CH_3)_2$<br>$-CH_2-CH-CH_2-$ | |
| 122 | 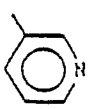 | 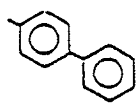 | O | O | $CH_3CHCH_2CH_3$<br>$-CH_2-CH-CH_2-$ | |
| 123 | 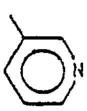 | 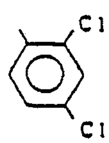 | O | O | $C(CH_3)_3$<br>$-CH_2-CH-CH_2-$ | |
| 124 | 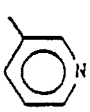 | 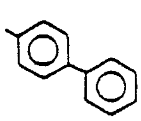 | O | O | $C(CH_3)_3$<br>$-CH_2-CH-CH_2-$ | |

Tabelle 1 (Fortsetzung)

| | | | | | | |
|---|---|---|---|---|---|---|
| 133 | 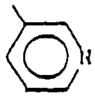 | 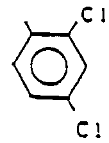 | o | o | 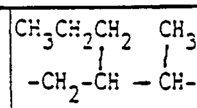 | |
| 134 | 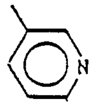 | 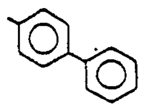 | o | o | 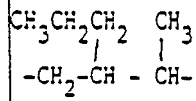 | |
| 135 | 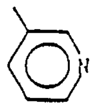 | 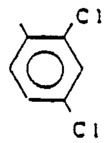 | o | o | 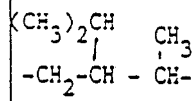 | |
| 136 | 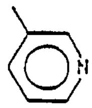 | 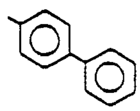 | o | o | 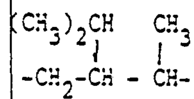 | |
| 137 | 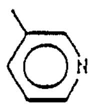 | 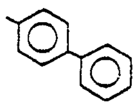 | o | o | 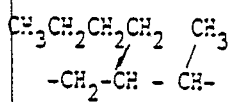 | |
| 138 | 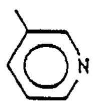 | 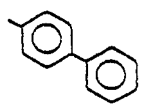 | o | o | 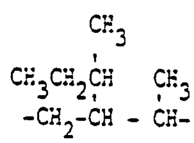 | |
| 139 | 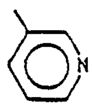 | 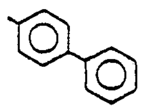 | o | o | 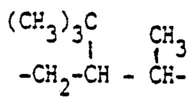 | |
| 140 | 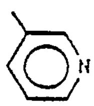 | 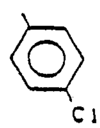 | o | o | 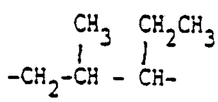 | |

Tabelle 1 (Fortsetzung)

| | | | | | | |
|---|---|---|---|---|---|---|
| 141 | 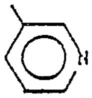 | 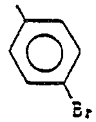 | 0 | 0 | $CH_3$ $CH_2CH_3$<br>$-CH_2-CH - CH-$ | |
| 142 | 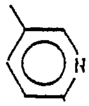 | 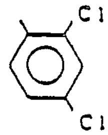 | 0 | 0 | $CH_3$ $CH_2CH_3$<br>$-CH_2-CH -CH-$ | |
| 143 | 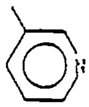 | 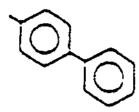 | 0 | 0 | $CH_3$ $CH_2CH_3$<br>$-CH_2-CH - CH-$ | |
| 144 | 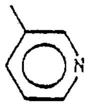 | 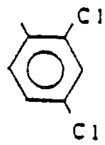 | 0 | 0 | $CH_3CH_2$ $CH_2CH_3$<br>$-CH_2-CH - CH-$ | |
| 145 | 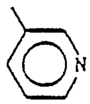 | 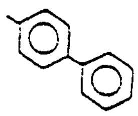 | 0 | 0 | $CH_3CH_2$ $CH_2CH_3$<br>$-CH_2-CH - CH-$ | |
| 146 | 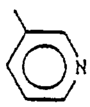 | 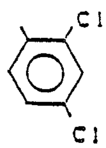 | 0 | 0 | $CH_3CH_2CH_2$ $CH_2CH_3$<br>$-CH_2-CH - CH-$ | |
| 147 | 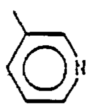 | 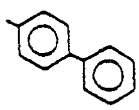 | 0 | 0 | $CH_3CH_2CH_2$ $CH_2CH_3$<br>$-CH_2-CH - CH-$ | |
| 148 | 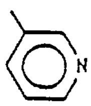 | 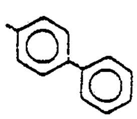 | 0 | 0 | $(CH_3)_2CH$ $CH_2CH_3$<br>$-CH_2-CH - CH-$ | |

Tabelle 1 (Fortsetzung)

| | | | | | | |
|---|---|---|---|---|---|---|
| 149 | 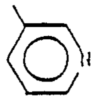 | 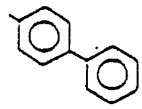 | 0 | 0 | $(CH_3)_3C$ $CH_2CH_3$<br>$-CH_2-CH$ $-CH-$ | |
| 150 | 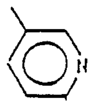 | 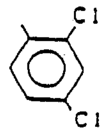 | 0 | 0 | $CH_3$ $CH_2CH_2CH_3$<br>$-CH_2-CH$ $-CH-$ | |
| 151 | 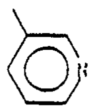 | 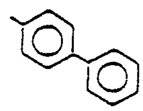 | 0 | 0 | $CH_3$ $CH_2CH_2CH_3$<br>$-CH_2-CH$ - $CH-$ | |
| 152 | 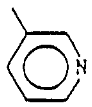 | 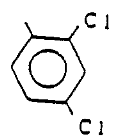 | 0 | 0 | $CH_3CH_2$ $CH_2CH_2CH_3$<br>$-CH_2-CH$ - $CH-$ | |
| 153 | 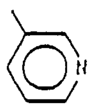 | 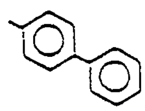 | 0 | 0 | $CH_3CH_2$ $CH_2CH_2CH_3$<br>$-CH_2-CH$ - $CH-$ | $n_D^{20}$ 1.5852 |
| 154 | 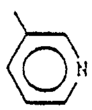 | 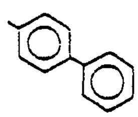 | 0 | 0 | $CH_3CH_2CH_2$ $CH_2CH_2CH_3$<br>$-CH_2-CH$ - $CH-$ | |
| 155 | 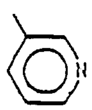 | 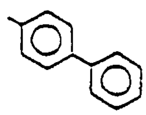 | 0 | 0 | $(CH_3)_2CH$ $CH_2CH_2CH_3$<br>$-CH_2-CH$ - $CH-$ | |
| 156 | 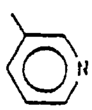 | 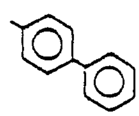 | 0 | 0 | $CH_3CH_2CH_2CH_2$ $CH_2CH_2CH_3$<br>$-CH_2-CH$ - $CH-$ | |

Tabelle 1 (Fortsetzung)

| | | | | | | |
|---|---|---|---|---|---|---|
| 157 | 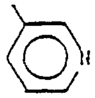 | 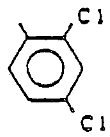 | 0 | 0 | $CH_3$ $CH(CH_3)_2$<br>$-CH_2-CH - CH-$ | |
| 158 | 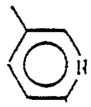 | 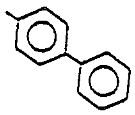 | 0 | 0 | $CH_3$ $CH(CH_3)_2$<br>$-CH_2-CH -CH-$ | |
| 159 | 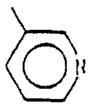 | 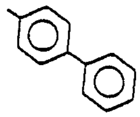 | 0 | 0 | $CH_3CH_2$ $CH(CH_3)_2$<br>$-CH_2-CH - CH-$ | |
| 160 | 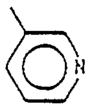 | 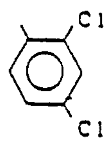 | 0 | 0 | $CH_3$ $CH_2CH_2CH_2CH_3$<br>$-CH_2-CH - CH-$ | |
| 161 | 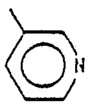 | 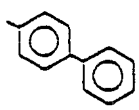 | 0 | 0 | $CH_3$ $CH_2CH_2CH_2CH_3$<br>$-CH_2-CH - CH-$ | |
| 162 | 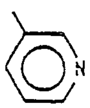 | 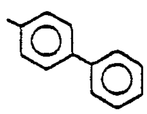 | 0 | 0 | $CH_3CH_2$ $CH_2CH_2CH_2CH_3$<br>$-CH_2-CH - CH-$ | |
| 163 | 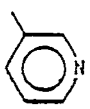 | 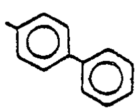 | 0 | 0 | $CH_3$<br>$CH_3$ $CHCH_2CH_3$<br>$-CH_2-CH -CH-$ | |
| 164 | 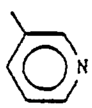 | 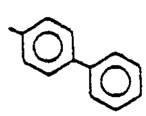 | 0 | 0 | $CH_3$ $C(CH_3)_3$<br>$-CH_2-CH - CH-$ | |

Tabelle 1 (Fortsetzung)

| | | | | | | |
|---|---|---|---|---|---|---|
| 165 | 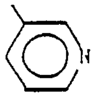 | 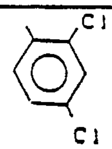 | 0 | 0 | $CH_3$ $CH_3$<br>$-CH-CH_2-CH-$ | |
| 166 | 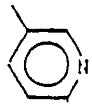 | 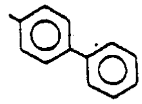 | 0 | 0 | $CH_3$ $CH_3$<br>$-CH-CH_2-CH-$ | $n_D^{20}$ 1.5967 |
| 167 | 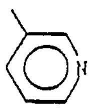 | 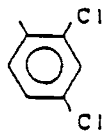 | 0 | 0 | $CH_3$ $CH_2CH_3$<br>$-CH-CH_2-CH-$ | |
| 168 | 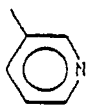 | 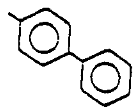 | 0 | 0 | $CH_3$ $CH_2CH_3$<br>$-CH-CH_2-CH-$ | |
| 169 | 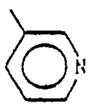 | 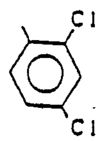 | 0 | 0 | $CH_3$ $CH_2CH_2CH_3$<br>$-CH-CH_2-CH-$ | |
| 170 | 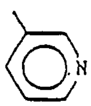 | 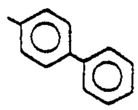 | 0 | 0 | $CH_3$ $CH_2CH_2CH_3$<br>$-CH-CH_2-CH-$ | |
| 171 | 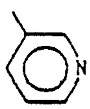 | 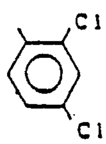 | 0 | 0 | $CH_3$ $CH(CH_3)_2$<br>$-CH-CH_2-CH-$ | |
| 172 | 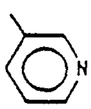 | 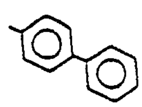 | 0 | 0 | $CH_3$ $CH(CH_3)_2$<br>$-CH-CH_2-CH-$ | |

Tabelle 1 (Fortsetzung)

| | | | | | | |
|---|---|---|---|---|---|---|
| 173 | 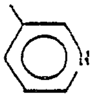 | 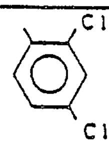 | 0 | 0 | $CH_3$ $CH_2CH_2CH_2CH_3$<br>$-CH-CH_2-CH-$ | |
| 174 | 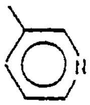 | 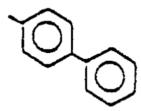 | 0 | 0 | $CH_3$ $CH_2CH_2CH_2CH_3$<br>$-CH-CH_2-CH-$ | |
| 175 | 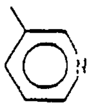 | 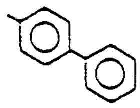 | 0 | 0 | $CH_3$ $CH_2CH(CH_3)_2$<br>$-CH-CH_2-CH-$ | |
| 176 | 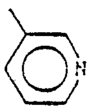 | 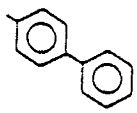 | 0 | 0 | $CH_3$<br>$CH_3$ $CHCH_2CH_3$<br>$-CH-CH_2-CH-$ | |
| 177 | 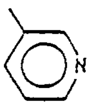 | 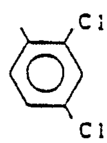 | 0 | 0 | $CH_3$ $C(CH_3)_3$<br>$-CH-CH_2-CH-$ | |
| 178 | 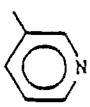 | 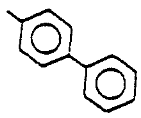 | 0 | 0 | $CH_3$ $C(CH_3)_3$<br>$-CH-CH_2-CH-$ | |
| 179 | 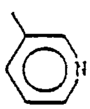 | 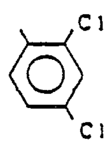 | 0 | 0 | $CH_3CH_2$ $CH_2CH_3$<br>$-CH-CH_2-CH-$ | |
| 180 | 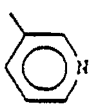 | 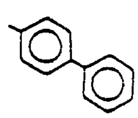 | 0 | 0 | $CH_3CH_2$ $CH_2CH_3$<br>$-CH-CH_2-CH-$ | |

Tabelle 1 (Fortsetzung)

| | | | | | | |
|---|---|---|---|---|---|---|
| 181 | 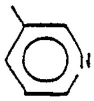 | 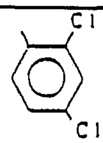 | 0 | 0 | 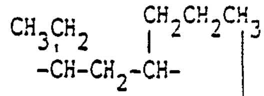 | |
| 182 | 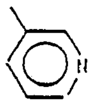 | 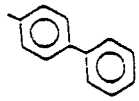 | 0 | 0 | 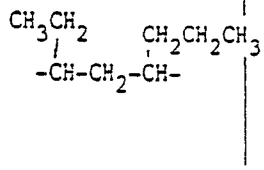 | |
| 183 | 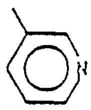 | 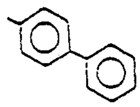 | 0 | 0 | 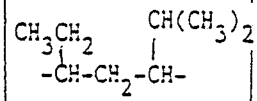 | |
| 184 | 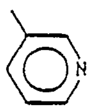 | 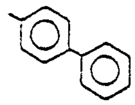 | 0 | 0 | 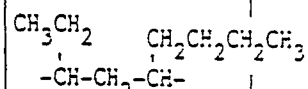 | |
| 185 | 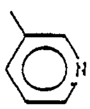 | 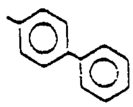 | 0 | 0 | 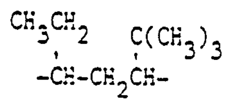 | |
| 186 | 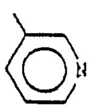 | 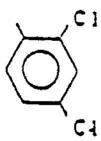 | 0 | 0 | 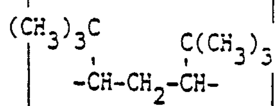 | |
| 187 | 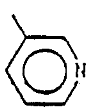 | 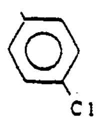 | 0 | 0 | 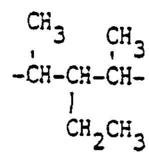 | |
| 188 | 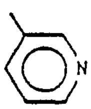 | 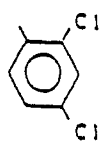 | 0 | 0 | 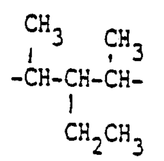 | |

Tabelle 1 (Fortsetzung)

| | | | | | | |
|---|---|---|---|---|---|---|
| 189 | 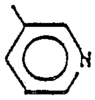 | 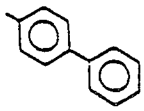 | 0 | 0 | 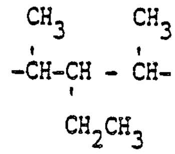 | |
| 190 | 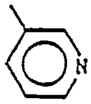 | 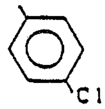 | 0 | 0 | 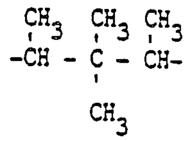 | |
| 191 | 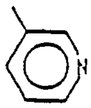 | 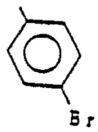 | 0 | 0 | 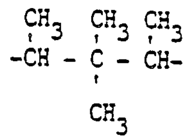 | |
| 192 | 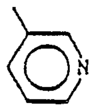 | 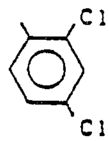 | 0 | 0 | 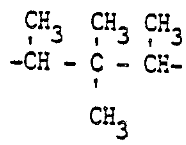 | |
| 193 | 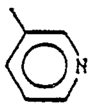 | 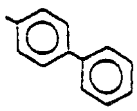 | 0 | 0 | 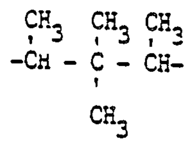 | |
| 194 | 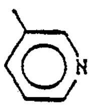 | 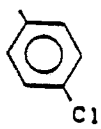 | 0 | 0 | 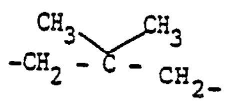 | $n_D^{20}$ 1.5659 |
| 195 | 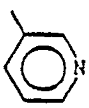 | 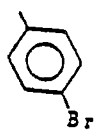 | 0 | 0 | 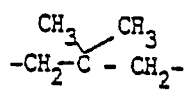 | |
| 196 | 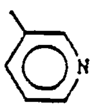 | 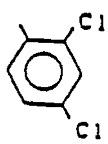 | 0 | 0 | 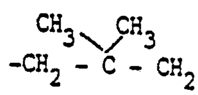 | $n_D^{20}$1.5684 |

Tabelle 1 (Fortsetzung)

| | | | | | | |
|---|---|---|---|---|---|---|
| 197 | 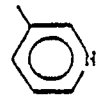 | 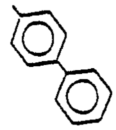 | O | O | $CH_3$ $CH_3$<br>$-CH_2-C-CH_2-$ | Schmp. 131 - 132.5 °C |
| 198 | 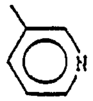 | 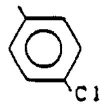 | O | O | $CH_3$ $CH_2CH_3$<br>$-CH_2-C-CH_2-$ | |
| 199 | 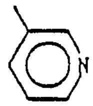 | 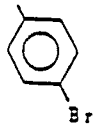 | O | O | $CH_3$ $CH_2CH_3$<br>$-CH_2-C-CH_2-$ | |
| 200 | 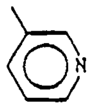 | 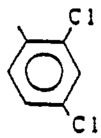 | O | O | $CH_3$ $CH_2CH_3$<br>$-CH_2-C-CH_2-$ | $n_D^{20}$ 1.5500 |
| 201 | 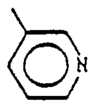 | 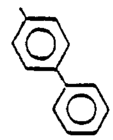 | O | O | $CH_3$ $CH_2CH_3$<br>$-CH_2-C-CH_2-$ | |
| 202 | 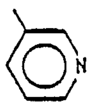 | 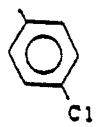 | O | O | $CH_3$ $CH_2CH_2CH_3$<br>$-CH_2-C-CH_2-$ | |
| 203 | 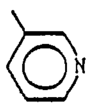 | 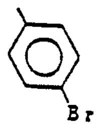 | O | O | $CH_3$ $CH_2CH_2CH_3$<br>$-CH_2-C-CH_2-$ | |
| 204 | 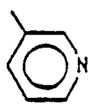 | 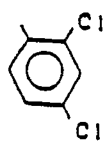 | O | O | $CH_3$ $CH_2CH_2CH_3$<br>$-CH_2-C-CH_2-$ | $n_D^{20}$ 1.5528 |

Tabelle 1 (Fortsetzung)

| | | | | | | |
|---|---|---|---|---|---|---|
| 205 | 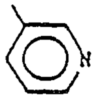 | 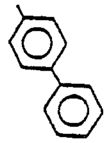 | 0 | 0 | $CH_3$ $CH_2CH_2CH_3$<br>$-CH_2-C-CH_2-$ | Schmp. 118 - 120°C |
| 206 | 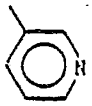 | 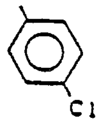 | 0 | 0 | $CH_3$ $CH(CH_3)_2$<br>$-CH_2-C-CH_2-$ | |
| 207 | 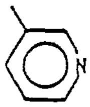 | 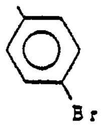 | 0 | 0 | $CH_3$ $CH(CH_3)_2$<br>$-CH_2-C-CH_2$ | |
| 208 | 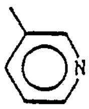 | 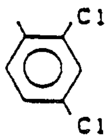 | 0 | 0 | $CH_3$ $CH(CH_3)_2$<br>$-CH_2-C-CH_2-$ | |
| 209 | 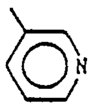 | 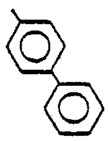 | 0 | 0 | $CH_3$ $CH(CH_3)_2$<br>$-CH_2-C-CH_2-$ | |
| 210 | 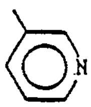 | 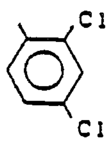 | 0 | 0 | $CH_3$ $CH_2CH_2CH_2CH_3$<br>$-CH_2-C-CH_2-$ | |
| 211 | 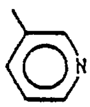 | 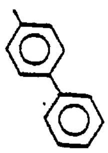 | 0 | 0 | $CH_3$ $CH_2CH_2CH_2CH_3$<br>$-CH_2-C-CH_2-$ | |
| 212 | 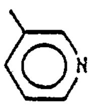 | 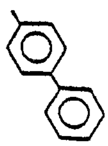 | 0 | 0 | $CH_3$ $CH_2CH(CH_3)_2$<br>$-CH_2-C-CH_2-$ | |

Tabelle 1 (Fortsetzung)

| | | | | | | |
|---|---|---|---|---|---|---|
| 213 | 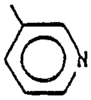 | 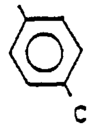 | 0 | 0 | 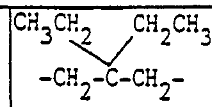 | $n_D^{20}$ 1.5642 |
| 214 | 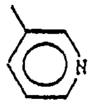 | 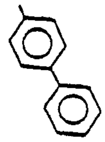 | 0 | 0 | 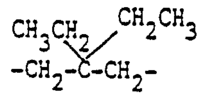 | Schmp. 125 - 127°C |
| 215 | 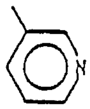 | 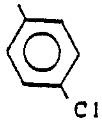 | 0 | 0 | 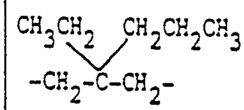 | |
| 216 | 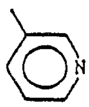 | 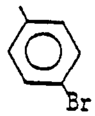 | 0 | 0 | 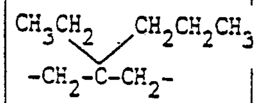 | |
| 217 | 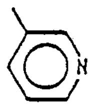 | 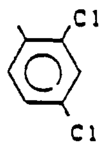 | 0 | 0 | 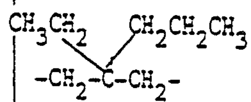 | $n_D^{20}$ 1.5425 |
| 218 | 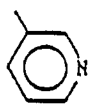 | 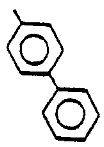 | 0 | 0 | 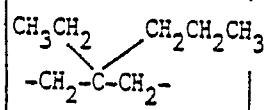 | |
| 219 | 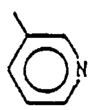 | 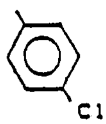 | 0 | 0 | 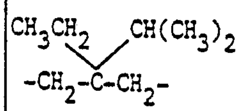 | |
| 220 | 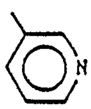 | 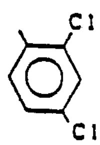 | 0 | 0 | 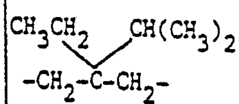 | |

| | | | | | | |
|---|---|---|---|---|---|---|
| 221 | 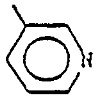 | 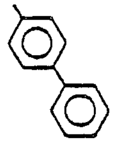 | O | O | 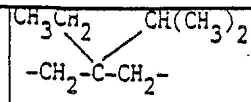 | |
| 222 | 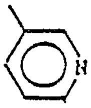 | 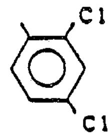 | O | O | 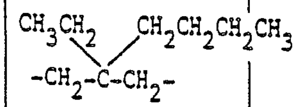 | |
| 223 | 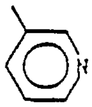 | 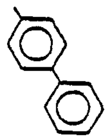 | O | O | 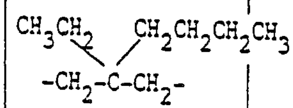 | |
| 224 | 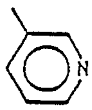 | 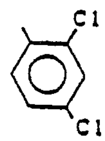 | O | O | 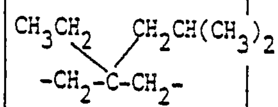 | |
| 225 | 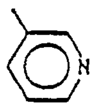 | 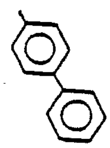 | O | O | 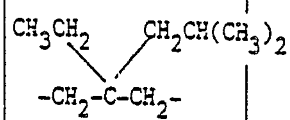 | |
| 226 | 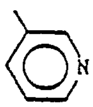 | 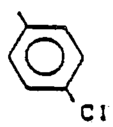 | O | O | 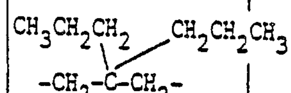 | |
| 227 | 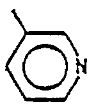 | 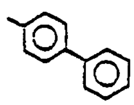 | O | O | 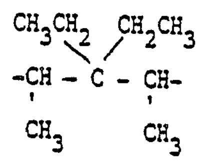 | |
| 228 | 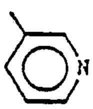 | 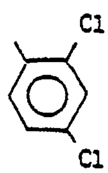 | S | O | $-CH_2CH_2-$ | |

Tabelle 1 (Fortsetzung)

| | | | | | | |
|---|---|---|---|---|---|---|
| 229 | 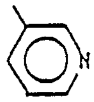 | 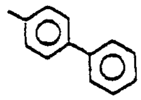 | S | O | $-CH_2CH_2-$ | |
| 230 | 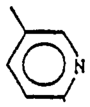 | 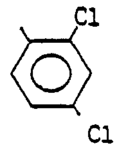 | S | O | $CH_3$<br>$-CH_2CH-$ | |
| 231 | 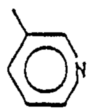 | 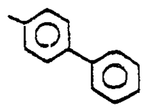 | S | O | $CH_3$<br>$-CH_2CH-$ | |
| 232 | 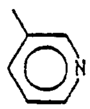 | 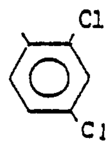 | S | O | $CH_3$ $CH_3$<br>$-CH - CH -$ | |
| 233 | 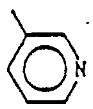 | 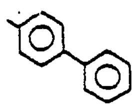 | S | O | $CH_3$ $CH_3$<br>$-CH - CH-$ | |
| 234 | 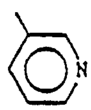 | 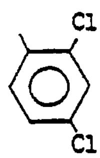 | S | O<br>‖<br>-S- | $-CH_2CH_2-$ | |
| 235 | 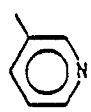 | 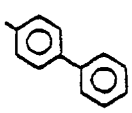 | S | O<br>‖<br>-S- | $-CH_2CH_2-$ | |
| 236 | 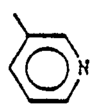 | 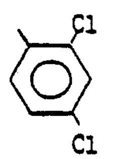 | S | O<br>‖<br>-S- | $CH_3$<br>$-CH_2CH-$ | |

Tabelle 1 (Fortsetzung)

| | | | | | | |
|---|---|---|---|---|---|---|
| 237 | 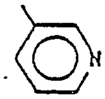 | 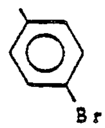 | O | O | 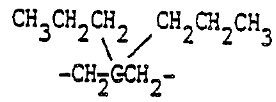 | |
| 238 | 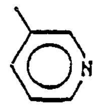 | 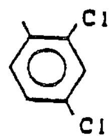 | O | O | 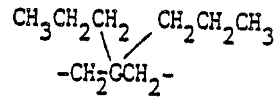 | |
| 239 | 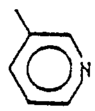 | 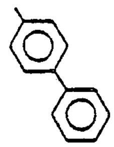 | O | O | 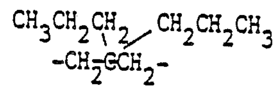 | |
| 240 | 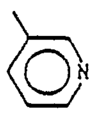 | 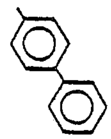 | O | O | 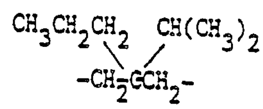 | |
| 241 | 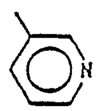 | 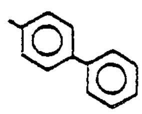 | O | O | 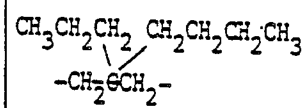 | |
| 242 | 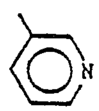 | 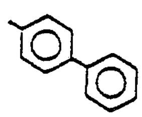 | O | O | 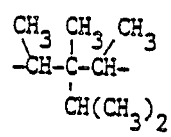 | |
| 243 | 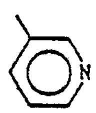 | 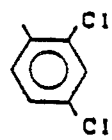 | O | O | 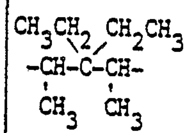 | |
| 244 | 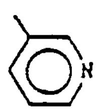 | 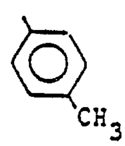 | S | S | 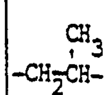 | ölig |

Tabelle 1 (Fortsetzung)

| | | | | | | |
|---|---|---|---|---|---|---|
| 245 | 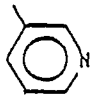 | 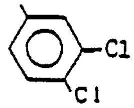 | S | S | $CH_3$<br>$-CH_2-CH-$ | |
| 246 | 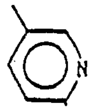 | 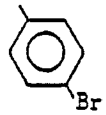 | S | S | $-CH_2-CH_2-$ | |
| 247 | 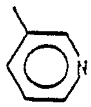 | 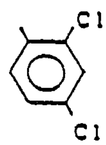 | S | S | $-CH_2-CH_2-$ | |
| 248 | 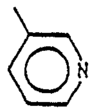 | 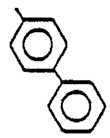 | S | S | $-CH_2-CH_2-$ | |
| 249 | 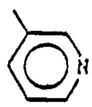 | 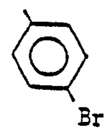 | S | S | $CH_3$<br>$-CH_2-CH-$ | |
| 250 | 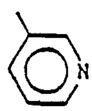 | 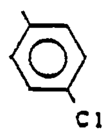 | S | S | $CH_2CH_3$<br>$-CH_2-CH-$ | |
| 251 | 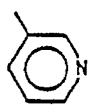 | 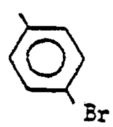 | S | S | $CH_2CH_3$<br>$-CH_2-CH-$ | |
| 252 | 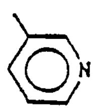 | 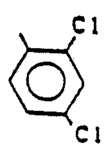 | S | S | $CH_2CH_3$<br>$-CH_2-CH-$ | |

Tabelle 1 (Fortsetzung)

| | | | | | | |
|---|---|---|---|---|---|---|
| 253 | 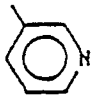 | 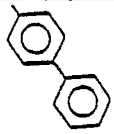 | S | S | $CH_2CH_3$<br>$-CH_2CH-$ | |
| 254 | 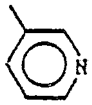 | 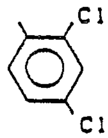 | S | S | $CH_2CH_2CH_3$<br>$-CH_2CH-$ | |
| 255 | 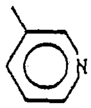 | 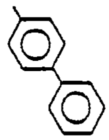 | S | S | $CH_2CH_2CH_3$<br>$-CH_2CH-$ | |
| 256 | 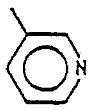 | 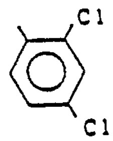 | S | S | $CH(CH_3)_2$<br>$-CH_2CH$ | |
| 257 | 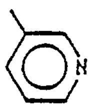 | 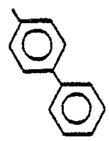 | S | S | $CH(CH_3)_2$<br>$-CH_2CH-$ | |
| 258 | 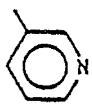 | 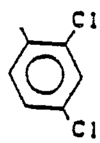 | S | S | $CH_2CH_2CH_2CH_3$<br>$-CH_2CH-$ | |
| 259 | 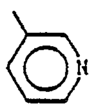 | 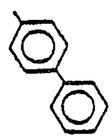 | S | S | $CH_2CH_2CH_2CH_3$<br>$-CH_2CH-$ | |
| 260 | 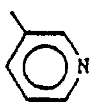 | 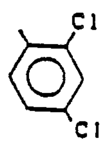 | S | S | $CH_2CH(CH_3)_2$<br>$-CH_2CH-$ | |

Tabelle 1 (Fortsetzung)

| | | | | | | |
|---|---|---|---|---|---|---|
| 261 | 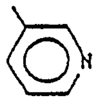 | 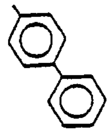 | S | S | $CH_2CH(CH_3)_2$<br>$-CH_2CH-$ | |
| 262 | 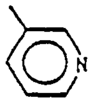 | 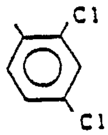 | S | S | $CH_3CHCH_2CH_3$<br>$-CH_2CH-$ | |
| 263 | 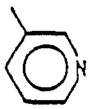 | 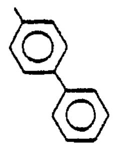 | S | S | $CH_3CHCH_2CH_3$<br>$-CH_2CH-$ | |
| 264 | 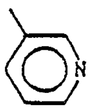 | 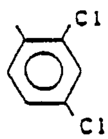 | S | S | $C(CH_3)_3$<br>$-CH_2CH-$ | |
| 265 | 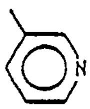 | 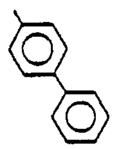 | S | S | $C(CH_3)_3$<br>$-CH_2CH-$ | |
| 266 | 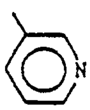 | 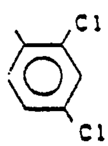 | S | S | $CH_2Cl$<br>$-CH_2CH-$ | |
| 267 | 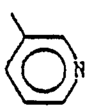 | 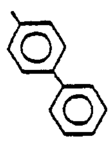 | S | S | $CH_2Cl$<br>$-CH_2CH-$ | |
| 268 | 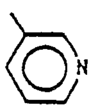 | 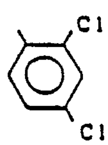 | S | S | $CH_2OCH_3$<br>$-CH_2CH-$ | |

Tabelle 1 (Fortsetzung)

| | | | | | | |
|---|---|---|---|---|---|---|
| 269 | 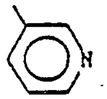 | 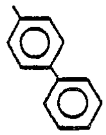 | S | S | $CH_2OCH_3$<br>$-CH_2CH-$ | |
| 270 | 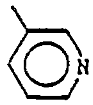 | 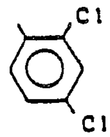 | S | S | 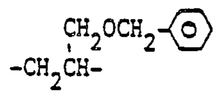 | |
| 271 | 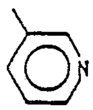 | 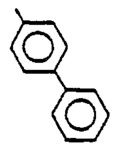 | S | S | 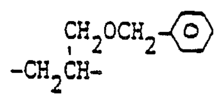 | |
| 272 | 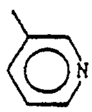 | 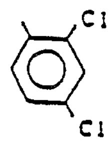 | S | S | $CH_3$ $CH_3$<br>-CHCH- | |
| 273 | 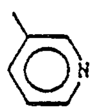 | 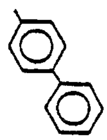 | S | S | $CH_3$ $CH_3$<br>-CHCH- | |
| 274 | 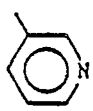 | 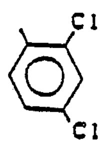 | S | S | $CH_3$ $CH_2CH_3$<br>-CHCH- | |
| 275 | 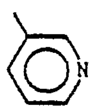 | 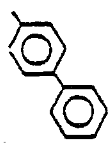 | S | S | $CH_3$ $CH_2CH_3$<br>-CHCH- | |
| 276 | 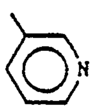 | 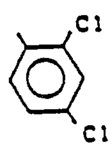 | S | S | $CH_3$ $CH_2CH_2CH_3$<br>-CHCH- | |

Tabelle 1 (Fortsetzung)

| | | | | | | |
|---|---|---|---|---|---|---|
| 277 | 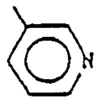 | 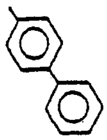 | S | S | $CH_3\ CH_2CH_2CH_3$<br>-CHCH- | |
| 278 | 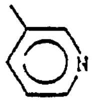 | 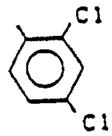 | S | S | $CH_3\ CH(CH_3)_2$<br>-CHCH- | |
| 279 | 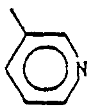 | 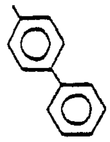 | S | S | $CH_3\ CH(CH_3)_2$<br>-CHCH- | |
| 280 | 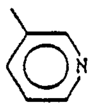 | 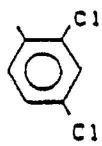 | S | S | $CH_3\ CH_2CH_2CH_2CH_3$<br>-CHCH- | |
| 281 | 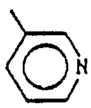 | 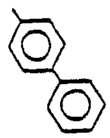 | S | S | $CH_3\ CH_2CH_2CH_2CH_3$<br>-CHCH- | |
| 282 | 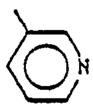 | 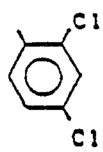 | S | S | $CH_3CH_2\ CH_2CH_3$<br>-CHCH- | |
| 283 | 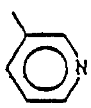 | 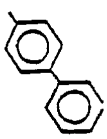 | S | S | $CH_3CH_2\ CH_2CH_3$<br>-CHCH- | |
| 284 | 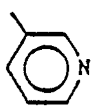 | 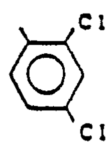 | S | S | $CH_3CH_2CH_2\ CH_2CH_2CH_3$<br>-CHCH- | |

Tabelle 1 (Fortsetzung)

| | | | | | | |
|---|---|---|---|---|---|---|
| 285 | 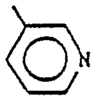 | 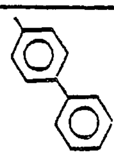 | S | S | $CH_3CH_2CH_2$ $CH_2CH_2CH_3$<br>\ /<br>-CHCH- | |
| 286 | 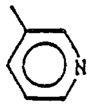 | 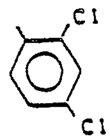 | S | S | $(CH_3)_2CH$ $CH(CH_3)_2$<br>\ \|<br>-CHCH- | |
| 287 | 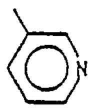 | 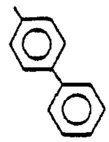 | S | S | $(CH_3)_2CH$ $CH(CH_3)_2$<br>\ \|<br>-CHCH- | |
| 288 | 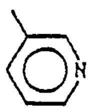 | 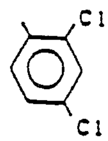 | S | S | $CH_3CH_2CH_2CH_2$ $CH_2CH_2CH_2CH_3$<br>\ /<br>-CHCH- | |
| 289 | 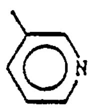 | 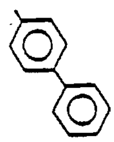 | S | S | $CH_3CH_2CH_2CH_2$ $CH_2CH_2CH_2CH_3$<br>\ /<br>-CHCH- | |
| 290 | 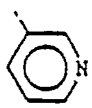 | 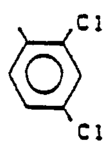 | S | S | $CH_3$ $CH_2Cl$<br>\ /<br>-CHCH- | |
| 291 | 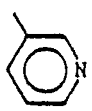 | 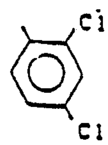 | S | S | $-CH_2CH_2CH_2-$ | |
| 292 | 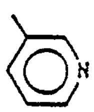 | 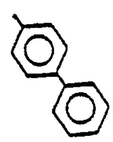 | S | S | $-CH_2CH_2CH_2-$ | |

Tabelle 1 (Fortsetzung)

| | | | | | | |
|---|---|---|---|---|---|---|
| 293 | 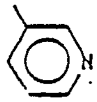 | 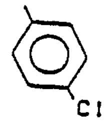 | S | S | $CH_3$<br>$-CH_2C-CH_2-$<br>$CH_3$ | |
| 294 | 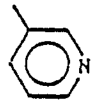 | 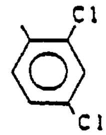 | S | S | $CH_3$<br>$-CH_2C-CH_2-$<br>$CH_3$ | |
| 295 | 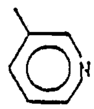 | 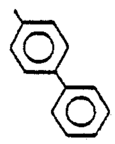 | S | S | $CH_3$<br>$-CH_2C-CH_2-$<br>$CH_3$ | |
| 296 | 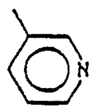 | 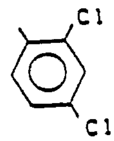 | O | O | $CH_3$ $CH_3$<br>$-CH_2-C-CH-$<br>$CH_3$ | |
| 297 | 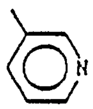 | 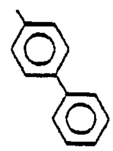 | O | O | $CH_3$ $CH_3$<br>$-CH_2-C-CH-$<br>$CH_3$ | |
| 298 | 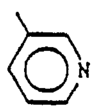 | 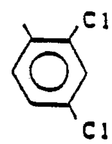 | O | O | $CH_3$ $CH_3$<br>$-CH_2-C-CH-$<br>$CH_2CH_3$ | |
| 299 | 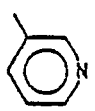 | 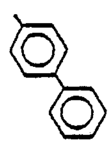 | O | O | $CH_3$ $CH_3$<br>$-CH_2-C-CH-$<br>$CH_2CH_3$ | |
| 300 | 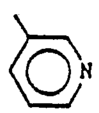 | 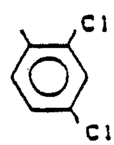 | O | O | $CH_3$ $CH_3$<br>$-CH_2-C-CH-$<br>$CH_2CH_2CH_3$ | |

Tabelle 1 (Fortsetzung)

| | | | | | | |
|---|---|---|---|---|---|---|
| 301 | 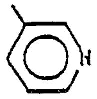 | 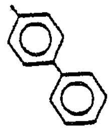 | 0 | 0 | 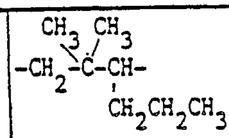 | |
| 302 | 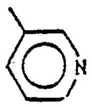 | 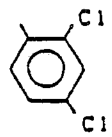 | 0 | 0 | 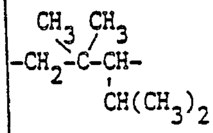 | |
| 303 | 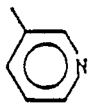 | 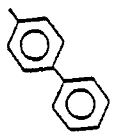 | 0 | 0 | 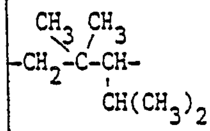 | |
| 304 | 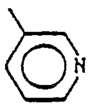 | 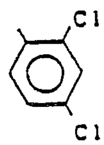 | 0 | 0 | 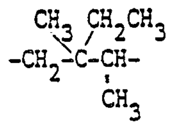 | |
| 305 | 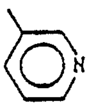 | 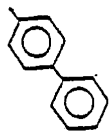 | 0 | 0 | 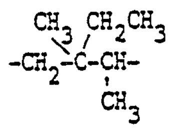 | |
| 306 | 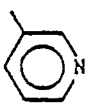 | 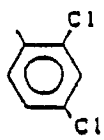 | 0 | 0 | 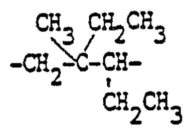 | |
| 307 | 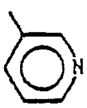 | 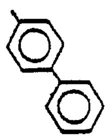 | 0 | 0 | 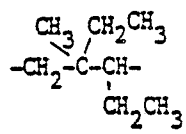 | |
| 308 | 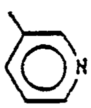 | 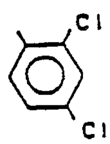 | 0 | 0 | 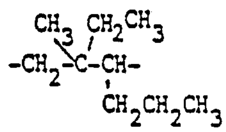 | |

Tabelle 1 (Fortsetzung)

| | | | | | | |
|---|---|---|---|---|---|---|
| 309 | 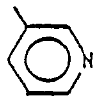 | 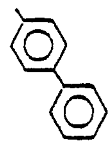 | O | O | $CH_3$ $CH_2CH_3$<br>-$CH_2$-C-CH-<br>$CH_2CH_2CH_3$ | |
| 310 | 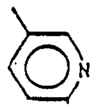 | 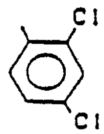 | O | O | $CH_3$ $CH_2CH_3$<br>-$CH_2$-C-CH-<br>$CH(CH_3)_2$ | |
| 311 | 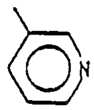 | 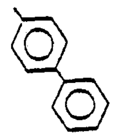 | O | O | $CH_3$ $CH_2CH_3$<br>-$CH_2$-C-CH-<br>$CH(CH_3)_2$ | |
| 312 | 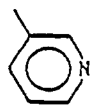 | 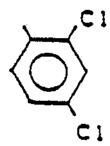 | O | O | $CH_3$ $CH_2CH_2CH_3$<br>-$CH_2$-C-CH-<br>$CH_3$ | |
| 313 | 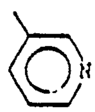 | 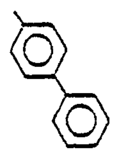 | O | O | $CH_3$ $CH_2CH_2CH_3$<br>-$CH_2$-C-CH-<br>$CH_3$ | |
| 314 | 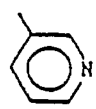 | 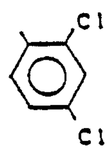 | O | O | $CH_3$ $CH_2CH_2CH_3$<br>-$CH_2$-C-CH-<br>$CH_2CH_3$ | |
| 315 | 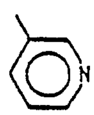 | 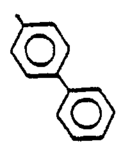 | O | O | $CH_3$ $CH_2CH_2CH_3$<br>-$CH_2$-C-CH-<br>$CH_2CH_3$ | |
| 316 | 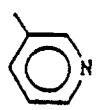 | 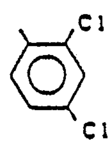 | O | O | $CH_3$ $CH(CH_3)_2$<br>-$CH_2$-C-CH-<br>$CH_3$ | |

Tabelle 1 (Fortsetzung)

| | | | | | | |
|---|---|---|---|---|---|---|
| 317 | 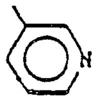 | 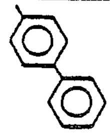 | 0 | 0 | 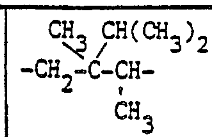 | |
| 318 | 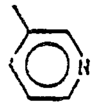 | 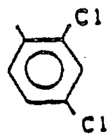 | 0 | 0 | 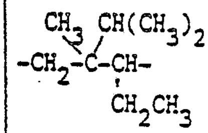 | |
| 319 | 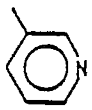 | 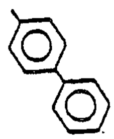 | 0 | 0 | 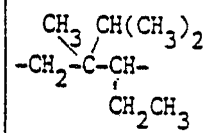 | |
| 320 | 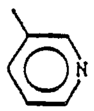 | 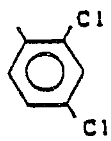 | 0 | 0 | 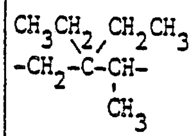 | |
| 321 | 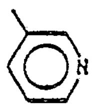 | 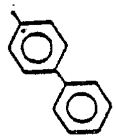 | 0 | 0 | 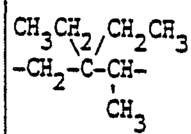 | |
| 322 | 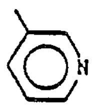 | 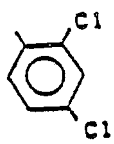 | 0 | 0 | 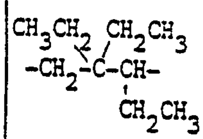 | |
| 323 | 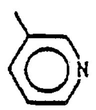 | 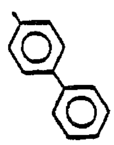 | 0 | 0 | 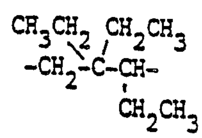 | |
| 324 | 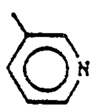 | 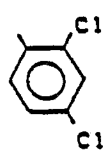 | 0 | 0 | 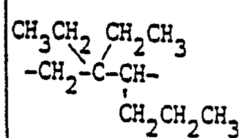 | |

Tabelle 1 (Fortsetzung)

| | | | | | | |
|---|---|---|---|---|---|---|
| 325 | 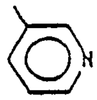 | 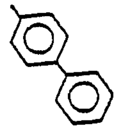 | 0 | 0 | 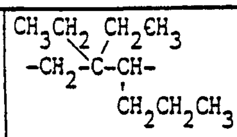 | |
| 326 | 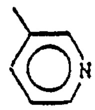 | 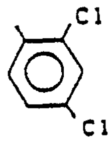 | 0 | 0 | 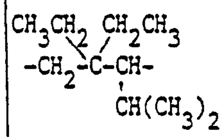 | |
| 327 | 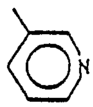 | 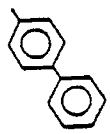 | 0 | 0 | 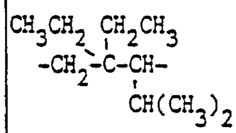 | |
| 328 | 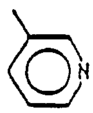 | 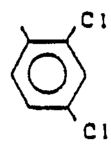 | 0 | 0 | 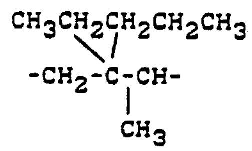 | |
| 329 | 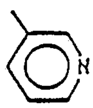 | 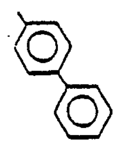 | 0 | 0 | 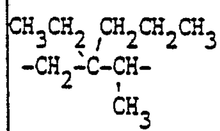 | |
| 330 | 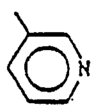 | 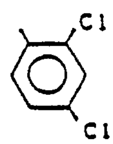 | 0 | 0 | 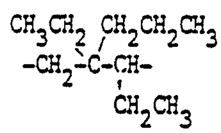 | |
| 331 | 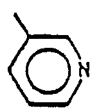 | 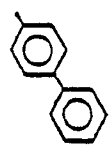 | 0 | 0 | 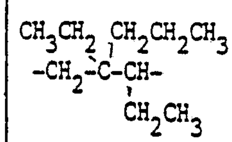 | |
| 332 | 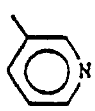 | 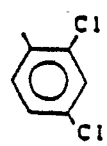 | 0 | 0 | 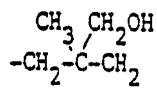 | |

Tabelle 1 (Fortsetzung)

| | | | | | | |
|---|---|---|---|---|---|---|
| 333 | 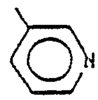 | 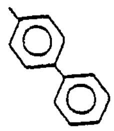 | O | O | 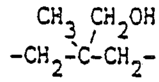 | |
| 334 | 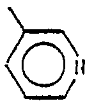 | 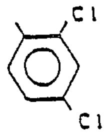 | O | O | 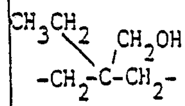 | |
| 335 | 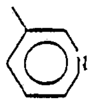 | 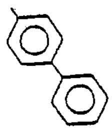 | O | O | 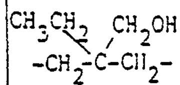 | |
| 336 | 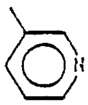 | 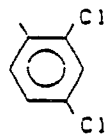 | O | O | 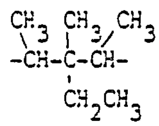 | |
| 337 | 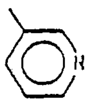 | 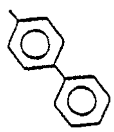 | O | O | 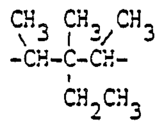 | |
| 338 | 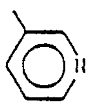 | 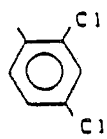 | O | O | 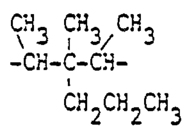 | |
| 339 | 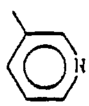 | 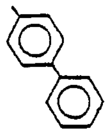 | O | O | 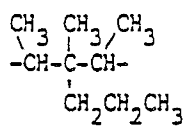 | |
| 340 | 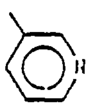 | 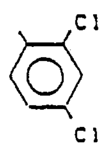 | O | O | 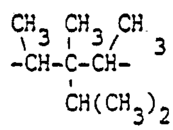 | |

Beispiel 6: Herstellung eines Zwischenprodukts

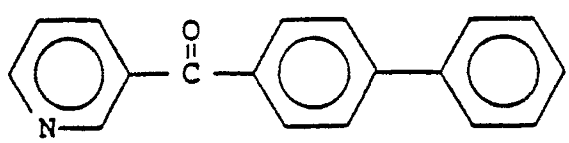

Zu einer Suspension von 17,8 g Nicotinylchlorid-hydrochlorid, 38,2 g Diphenyl und 100 ml Chlorbenzol

wurden 33,3 g Aluminiumchlorid unter Eiskühlung hinzugefügt, und die resultierende Mischung wurde 6 h unter Rückfluß erhitzt. Nach dem Abkühlen wurde die Reaktionsmischung in ein Gemisch aus Eis/Salzsäure gegossen, und das Gemisch wurde mit Chloroform gewaschen. Die wäßrige Schicht wurde mit einer wäßrigen Natriumhydroxid-Lösung alkalisch gemacht und mit Chloroform extrahiert. Die organische Schicht wurde mit Wasser gewaschen und über wasserfreiem Natriumsulfat getrocknet. Nach dem Entfernen des Natriumsulfats wurde das Lösungsmittel unter vermindertem Druck abdestilliert, wonach 9,1 g 3-(4-Phenylbenzoyl)pyridin erhalten wurden; Schmp. 112-115 °C.

Beispiel 7: Herstellung eines Zwischenprodukts

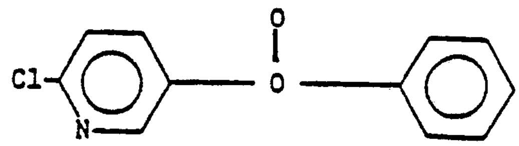

Zu 40 ml einer Lösung von 5,28 g 6-Chloronicotinylchlorid in Benzol wurden 16 g Aluminiumchlorid unter Eiskühlung hinzugefügt, und die resultierende Mischung wurde 6 h unter Rückfluß erhitzt. Nach dem Abkühlen wurde die Reaktionsmischung in ein Gemisch aus Eis/Salzsäure gegossen, und das Gemisch wurde mit Ether extrahiert. Die organische Phase wurde mit Wasser gewaschen und über wasserfreiem Natriumsulfat getrocknet. Nach dem Abfiltrieren des Natriumsulfats und dem Abdestillieren des Lösungsmittels wurden 4,6 g 3-Benzoyl-6-chloropyridin erhalten; Schmp. 39-40 °C.

Die folgenden Verbindungen können nach dem gleichen Verfahren, wie es im Vorstehenden beschrieben ist, hergestellt werden:

3-Benzoyl-5-chloropyridin

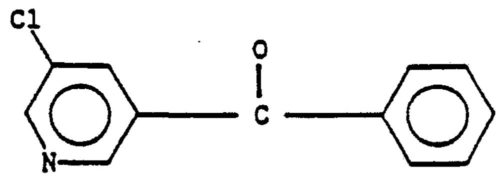

(Schmp. 74-75 °C)

und

3-Benzoyl-5,6-dichloropyridin

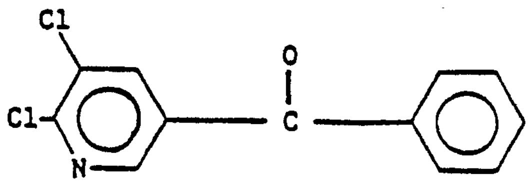

(Schmp. 88-89 °C).

Biotest-Beispiele:

Eingesetzte bekannte Vergleichs-Verbindungen:

E-1

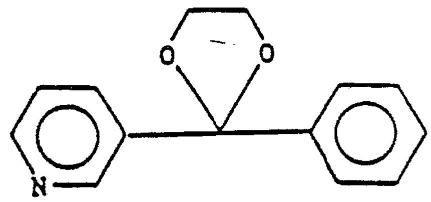

{Bull. Soc. Chim. Belg. 89, 67 (1980)}

E-2

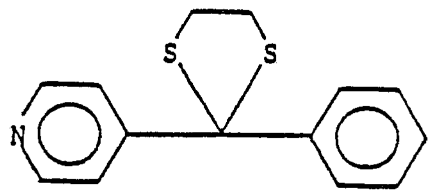

{J. Chem. Soc. Perkin Trans. I 1984, 1223}

E-3

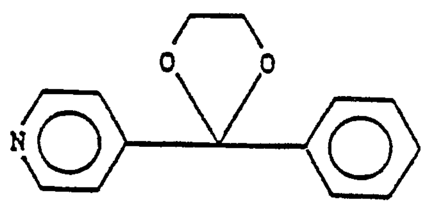

{Heterocycles 22, 1137 (1984)}

Beispiel 8

Test zur Bestimmung der Wirksamkeit gegen Helminthosporium-Fleckenkrankheit (die aktiven Verbindungen wurden auf Stengel und Blätter gesprüht)

| Formulierung der aktiven Verbindungen | |
|---|---|
| Zu testende Verbindung:<br>Organisches Lösungsmittel (Xylol): | 30 Teile<br>55 Teile |
| Emulgator: | |
| Polyoxyethylenalkylphenylether<br>Calciumalkylbenzolsulfonat | 8 Teile<br>7 Teile |

Die vorgeschriebene Dosis der wie vorstehend erhaltenen Emulsion wird dann durch Verdünnen mit Wasser hergestellt.

Test-Verfahren:

Reispflanzen (Varietät: Kusabue) wurden in Porzellan-Töpfen mit einem Durchmesser von 12 cm gezogen. Auf die Reispflanzen im 3- bis 4-Blatt-Stadium wurde das Präparat der aktiven Verbindung in einer Menge von 50 ml pro jeweils drei Töpfe aufgesprüht. Am nächsten Tag wurden die Pflanzen zweimal mit einer Sporen-Suspension des Gomahagarebyo verursachenden Pilzes besprüht, die künstlich kultiviert worden war, und 24 h in einer Feuchtigkeits-Kammer mit einer relativen Luftfeuchtigkeit von 100% und einer Temperatur von 25 °C gehalten. Nach der künstlichen Inokulierung wurden die Pflanzen in das Gewächshaus mit 22 °C bis 30 °C gestellt.

Sieben Tage nach der Inokulierung wurde die Infektion der Reispflanzen bestimmt und entsprechend der nachstehenden Bewertungs-Skala bewertet.

| Bewertung der Infektion | Grad der Infektion |
|---|---|
| 0 | keine |
| 1 | sehr geringfügig |
| 2 | geringfügig |
| 3 | mittel |
| 4 | stark |
| 5 | sehr ernst |

Der prozentuale Schutz wurde nach der folgenden Gleichung berechnet:

$$\text{Schutz (\%)} = \frac{A - B}{A} \times 100 \quad ;$$

hierin bezeichnet A den Grad der Infektion in dem (unbehandelten) Kontroll-Abschnitt, und B bezeichnet den Grand der Infektion in dem behandelten Abschnitt.

Die Ergebnisse dieses Tests sind in Tabelle 2 angegeben.

Tabelle 2

| Verbindung Nr. | Wirkstoff-Konzentration ppm | Schutz % |
|---|---|---|
| 1 | 500 | 100 |
| 2 | 500 | 100 |
| 3 | 500 | 100 |
| 10 | 500 | 100 |
| 16 | 500 | 100 |
| 17 | 500 | 100 |
| 19 | 500 | 100 |
| 20 | 500 | 100 |
| 22 | 500 | 100 |
| 31 | 500 | 100 |
| 32 | 500 | 100 |
| 35 | 500 | 100 |
| 37 | 500 | 100 |
| 41 | 500 | 100 |
| Vergleich | | |
| E-1 | 500 | 47 |
| E-2 | 500 | 60 |
| E-3 | 500 | 43 |

Beispiel 9

Test zur Bestimmung der Wirksamkeit der Bekämpfung von Gurken-Mehltau

Test-Verfahren

Eine Test-Verbindung in Form einer Emulsion, die nach Beispiel 8 hergestellt worden war, wurde mit Hilfe einer Spritzpistole auf Gurken-Pflanzen (Varietät: Tokiwajibai) im 2-Blatt-Stadium gesprüht, die in porösen Töpfen von 9 cm Durchmesser gezogen worden waren. Einen Tag nach dem Sprühen wurde eine Suspension von Sporen des Pathogens (Sphaerotheca fuliginea) durch Sprühen inokuliert. Nach Aufbewahren in einem thermostatisierten Raum von 23 °C wurde am zehnten Tag der Grad der Infektion auf der Basis der Fläche der Läsion bestimmt, um die Bekämpfungswirkung zu berechnen.

| Grad der Infektion | Anteil der Fläche der Verletzungen (%) |
|---|---|
| 0 | 0 |
| 0,5 | weniger als 2 |
| 1 | 3 bis 5 |
| 2 | 6 bis 15 |
| 3 | 16 bis 30 |
| 4 | 31 bis 50 |
| 5 | mehr als 51 |

$$\text{Bekämpfungs-Wert (\%)} = \frac{\left[\text{Grad der Infektion der unbehandelten Fläche}\right] - \left[\text{Grad der Infektion der behandelten Fläche}\right]}{\text{Grad der Infektion der unbehandelten Fläche}} \times 100$$

Die Ergebnisse dieses Tests sind in Tabelle 3 angegeben.

Tabelle 3

| Verbindung Nr. | Wirkstoff-Konzentration ppm | Schutz % |
|---|---|---|
| 42 | 100 | 100 |
| 57 | 100 | 100 |
| 58 | 100 | 100 |
| 60 | 100 | 100 |
| 75 | 100 | 100 |
| 100 | 100 | 100 |
| 130 | 100 | 100 |
| 200 | 100 | 100 |
| 214 | 100 | 100 |
| Vergleich | | |
| E-1 | 100 | 20 |

Beispiel 10

Test zur Bestimmung der hebriziden Wirksamkeit / Wasser-Oberflächenbehandlung / Unkräuter auf Böden, wo Reispflanzen unter Bewässerungsbedingungen kultiviert werden

Formulierung der aktiven Verbindungen

| | |
|---|---|
| Träger: | 5 Gew.-Teile Aceton |
| Emulgator: | 1 Gew.-Teil Benzyloxypolyglycolether |

Zur Herstellung eines geeigneten Präparats der aktiven Verbindung wurde 1 Gew.-Teil der aktiven Verbindung mit der angegebene Menge Träger und der angegebenen Menge Emulgator vermischt, und das resultierende emulgierbare Konzentrat wird dann mit Wasser auf die gewünschte Konzentration verdünnt.

Test-Verfahren

Mehrere Töpfe, die jeweils eine Größe von 25 cm x 20 cm x 9 cm und eine Fläche von 5 $dm^2$ besaßen, wurden mit Boden gefüllt, der aus einem Wasser enthaltenden Reisfeld entnommen worden war. Reis-Setzlinge (Varietät: Nihonbare) im 2,5-Blattstadium mit einer mittleren Höhe von 15 cm wurden in diese Töpfe umgepflanzt. Jeder Topf hat zwei Zonen mit jeweils einem aus drei Pflanzen bestehenden Stock. Dann wurden in jeden der Töpfe, die unter Naß-Bedingungen gehalten wurden, Samen der folgenden

Pflanzen eingesät:

| | |
|---|---|
| Hühnerhirse | (Echinochloa), |
| Schlanke Ährenbinse | (Eleocharis) |
| Flachriedgras | (Cyperus), |
| Monochoria | (Monochoria) und |

jährliche breitblättrige Unkräuter wie

| | |
|---|---|
| Falsche Pimpernelle | (Lindernia), |
| Zahnkelch | (Rotala), |
| Wassersternwurz | (Elatine), |
| Rotstengel | (Ammania) und |
| Dopatrium | (Dopatrium). |

Nach zwei Tagen wurde jeder der Töpfe bis zu einer Tiefe von 2 bis 3 cm mit Wasser gefüllt.

Fünf Tage nach dem Umpflanzen der Reis-Setzlinge wurde die Emulsion der aktiven Verbindung, die in der oben angegebenen Weise hergestellt worden war, in einer vorher festgelegten Menge mit einer Pipette in jeden der Töpfe gegeben. Danach wurde die Wasser-Schicht auf einer Dicke von etwa 3 cm gehalten.

Vier Wochen nach der Anwendung der aktiven Verbindung wurde der Grad der Schädigung der Unkräuter und der Grad der Phytotoxizität gegenüber den Reispflanzen bestimmt und nach der folgenden Bewertungs-Skala bewertet.

| Bewertung | Herbizide Wirkung der aktiven Verbindung gegen Unkräuter in % *) |
|---|---|
| 5 | 95 % oder mehr (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| **Bewertung** | **Phytotoxische Wirkung der aktiven Verbindung gegen Nutzpflanzen in % *)** |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

*) Diese Werte (%) werden durch Vergleich der Test-Daten in dem behandelten Pflanzen-Abschnitt mit dem (unbehandelten) Kontrollpflanzen-Abschnitt erhalten.

Die Testergebnisse sind in Tabelle 4 aufgeführt.

Tabelle 4

| Aktive Verbindung | Menge der aktiven Verbindung kg/ha | Herbizide Wirkung gegen Unkräuter | | | | | Phytotoxisch. Effekt gegen Reis-Pflanzen |
|---|---|---|---|---|---|---|---|
| | | Hühnerhirse | Schlanke Ährenbinse | Flachriedgras | Monochoria | Jährl. breitblättr. Unkräuter | |
| 1 | 2 | 3 | 5 | 4 | 5 | 5 | 0 |
| 2 | 2 | 5 | 5 | 5 | 5 | 5 | 0 |
| 12 | 2 | 4 | 5 | 5 | 5 | 5 | 0 |
| 19 | 2 | 5 | 5 | 5 | 5 | 5 | 0 |
| 32 | 2 | 5 | 5 | 5 | 5 | 5 | 0 |
| Vergleich | | | | | | | |
| E-2 | 2 | 0 | 0 | 0 | 0 | 0 | 0 |
| E-3 | 2 | 0 | 0 | 0 | 0 | 0 | 0 |

## Ansprüche

1. 3-Substituierte Pyridine der Formel (I)

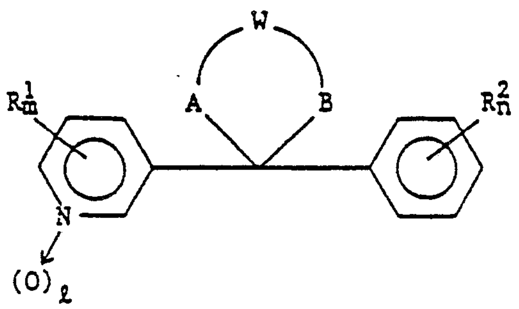

(I)

in der
A Sauerstoff oder Schwefel bezeichnet,
B Sauerstoff, Schwefel oder Sulfinyl bezeichnet,
$R^1$ Wasserstoff, Halogen, Alkyl oder Halogenoalkyl bezeichnet,
$R^2$ Wasserstoff, Halogen, Alkyl, Phenyl, Halogenoalkyl oder Phenoxy bezeichnet,
$\ell$ 0 oder 1 ist,
m eine ganze Zahl von 1 bis 4 ist,
n eine ganze Zahl von 1 bis 5 ist und
W eine Gruppe der Formel

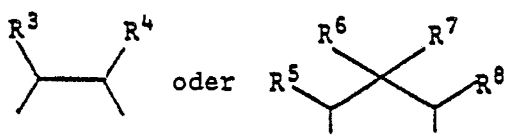

bezeichnet, worin
$R^3$ und $R^4$ jeweils Wasserstoff, Hydroxyalkyl, Alkoxyalkyl, Alkyl, Benzyloxyalkyl, Halogenoalkyl oder Carboxyalkyl bezeichnen oder
$R^3$ und $R^4$ zusammen mit den Kohlenstoff-Atomen, an die sie gebunden sind, einen Kohlenwasserstoff-Ring mit insgesamt 3 bis 12 Kohlenstoff-Atomen bilden und
$R^5$, $R^6$, $R^7$ und $R^8$ jeweils Wasserstoff, Alkyl oder Hydroxyalkyl bezeichnen,
mit der Maßgabe, daß keiner der Substituenten $R^1$, $R^2$, $R^3$ und $R^4$ ein Wasserstoff-Atom bezeichnet, wenn A und B jeweils ein Sauerstoff-Atom bezeichnen und W eine Gruppe der Formel

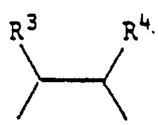

bezeichnet.

2. 3-Substituierte Pyridine nach Anspruch 1, dadurch gekennzeichnet, daß in der Formel (I)
A und B jeweils Sauerstoff oder Schwefel bezeichnen,
$R^1$ Wasserstoff oder Chlor bezeichnet,
$R^2$ Wasserstoff, Chlor, Brom, Alkyl mit 1 bis 4 Kohlenstoff-Atomen oder Phenyl bezeichnet,
m und n jeweils 1 oder 2 sind und
W eine Gruppe der Formel

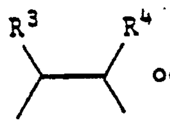 oder 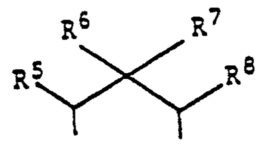

bezeichnet, worin

$R^3$ und $R^4$ jeweils Wasserstoff, Alkyl mit 1 bis 6 Kohlenstoff-Atomen, Hydroxyalkyl mit 1 bis 3 Kohlenstoff-Atomen, Alkoxyalkyl mit insgesamt 2 bis 4 Kohlenstoff-Atomen oder Halogenoalkyl mit 1 bis 4 Kohlenstoff-Atomen bezeichnen oder $R^3$ und $R^4$ zusammen mit den Kohlenstoff-Atomen, an die sie gebunden sind, einen Kohlenwasserstoff-Ring mit insgesamt 6 bis 12 Kohlenstoff-Atomen bilden und

$R^5$, $R^6$, $R^7$ und $R^8$ jeweils Wasserstoff, Alkyl mit 1 bis 4 Kohlenstoff-Atomen oder Hydroxyalkyl mit 1 bis 4 Kohlenstoff-Atomen bezeichnen,

mit der Maßgabe, daß keiner der Substituenten $R^1$, $R^2$, $R^3$ und $R^4$ Wasserstoff bezeichnet, wenn A und B jeweils Sauerstoff bezeichnen und W eine Gruppe der Formel

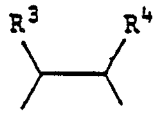

bezeichnet.

3. 3-Substituierte Pyridine nach Anspruch 1, dadurch gekennzeichnet, daß in der Formel (I)

A und B jeweils Sauerstoff oder Schwefel bezeichnen,

$R^1$ Wasserstoff oder Chlor bezeichnet,

$R^2$ Wasserstoff, Chlor, Brom , Methyl oder Phenyl bezeichnet,

m und n jeweils 1 oder 2 sind und

W eine Gruppe

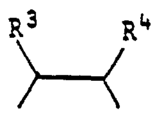 oder 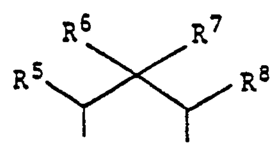

bezeichnet, worin

$R^3$ und $R^4$ jeweils Wasserstoff, Alkyl mit 1 bis 4 Kohlenstoff-Atomen, Methoxymethyl oder Chloromethyl bezeichnen oder

$R^3$ und $R^4$ zusammen mit den Kohlenstoff-Atomen, an die sie gebunden sind, einen Cyclohexan-Ring bilden und

$R^5$, $R^6$, $R^7$ und $R^8$ jeweils Wasserstoff oder Alkyl mit 1 bis 3 Kohlenstoff-Atomen bezeichnen,

mit der Maßgabe, daß keiner der Substituenten $R^1$, $R^2$, $R^3$ und $R^4$ ein Wasserstoff-Atom bezeichnet, wenn A und B jeweils Sauerstoff bezeichnen und W eine Gruppe der Formel

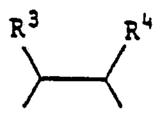

bezeichnet.

4. 2-Phenyl-2-(pyridin-3-yl)-1,3-dithiolan der Formel

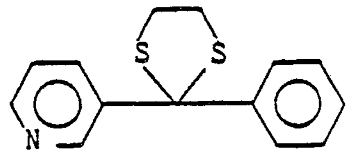

und
4-Methyl-2-phenyl-2-(pyridin-3-yl)-1,3-dithiolan der Formel

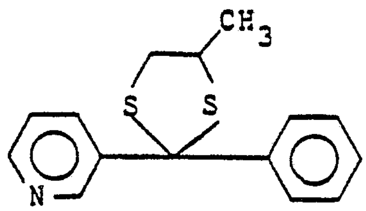

nach Anspruch 1.

5. Verfahren zur Herstellung 3-substituierten Pyridinen der Formel (I)

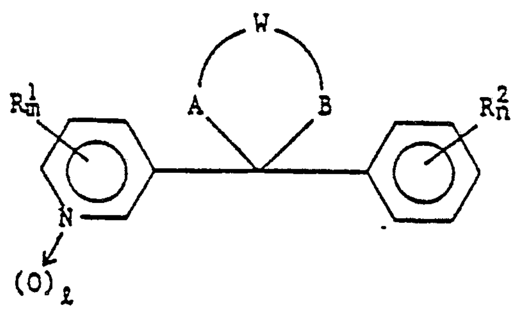

(I)

in der

A Sauerstoff oder Schwefel bezeichnet,
B Sauerstoff, Schwefel oder Sulfinyl bezeichnet,
$R^1$ Wasserstoff, Halogen, Alkyl oder Halogenoalkyl bezeichnet,
$R^2$ Wasserstoff, Halogen, Alkyl, Phenyl, Halogenoalkyl oder Phenoxy bezeichnet,
$\ell$ 0 oder 1 ist,
m eine ganze Zahl von 1 bis 4 ist,
n eine ganze Zahl von 1 bis 5 ist und
W eine Gruppe der Formel

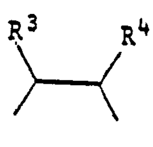

oder

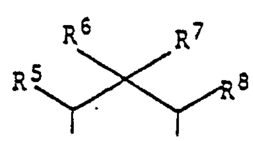

bezeichnet, worin
$R^3$ und $R^4$ jeweils Wasserstoff, Hydroxyalkyl, Alkoxyalkyl, Alkyl, Benzyloxyalkyl, Halogenoalkyl oder Carboxyalkyl bezeichnen oder
$R^3$ und $R^4$ zusammen mit den Kohlenstoff-Atomen, an die sie gebunden sind, einen Kohlenwasserstoff-Ring mit insgesamt 3 bis 12 Kohlenstoff-Atomen bilden und
$R^5$, $R^6$, $R^7$ und jeweils Wasserstoff, Alkyl oder Hydroxyalkyl bezeichnen,
mit der Maßgabe, daß keiner der Substituenten $R^1$, $R^2$, $R^3$ und $R^4$ ein Wasserstoff-Atom bezeichnet, wenn A und B jeweils ein Sauerstoff-Atom bezeichnen und W eine Gruppe der Formel

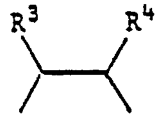

bezeichnet,
dadurch gekennzeichnet, daß

a) in dem Fall, in dem ℓ 0 ist und B Sauerstoff oder Schwefel bezeichnet,

Verbindungen der Formel (II)

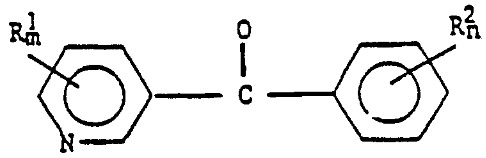

(II)

,

in der $R^1$, $R^2$, m und n die im Vorstehenden angegebenen Bedeutungen haben, mit Verbindungen der Formel (III)

HA - W - $B'$H (III),

in der A und W die im Vorstehenden angegebenen Bedeutungen haben und
$B'$ Sauerstoff oder Schwefel bezeichnet, in Gegenwart inerter Lösungsmittel und in Gegenwart von Säure-Katalysatoren umgesetzt werden,
oder

b) in dem Fall, in dem ℓ 1 ist und A und B jeweils Sauerstoff bezeichnen,

Verbindungen der Formel (Ib)

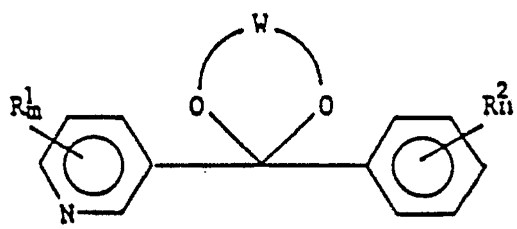

(Ib)

,

in der $R^1$, $R^2$, m, n und W die im Vorstehenden angegebenen Bedeutungen haben, mit einem Oxidationsmittel in Gegenwart inerter Lösungsmittel umgesetzt werden,
oder

c) in dem Fall, in dem B eine Sulfinyl-Gruppe bezeichnet und ℓ 0 ist,

Verbindungen der Formel (Ic)

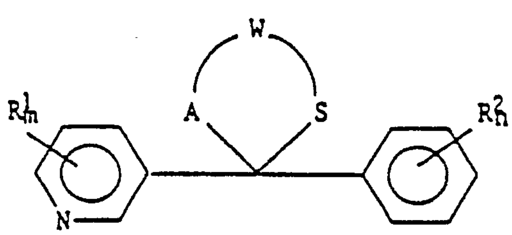

(Ic)

,

in der $R^1$, $R^2$, m, n, A und W die im Vorstehenden angegebenen Bedeutungen haben, mit einem Oxidationsmittel in Gegenwart inerter Lösungsmittel umgesetzt werden.

6. Fungizide und/oder herbizide Zusammensetzungen, dadurch gekennzeichnet, daß sie wenigstens ein 3-substituiertes Pyridin der Formel (I) nach den Ansprüchen 1 bis 5 enthalten.

7. Verfahren zur Bekämpfung von Fungi, dadurch gekennzeichnet, daß man 3-substituierte Pyridine der Formel (I) nach den Ansprüchen 1 bis 5 auf die Fungi und/oder deren Lebensraum einwirken läßt.

8. Verfahren zur Bekämpfung von Unkrautern, dadurch gekennzeichnet, daß man 3-substituierte Pyridine der Formel (I) nach den Ansprüchen 1 bis 5 auf die Unkräuter und/oder deren Lebensraum einwirken läßt.

9. Verwendung von 3-substituierten Pyridinen der Formel (I) nach den Ansprüchen 1 bis 5 zur Bekämpfung von Fungi und/oder Unkräutern.

10. Verfahren zur Herstellung von fungiziden und/oder herbiziden Zusammensetzungen, dadurch gekennzeichnet, daß 3-substituierte Pyridine der Formel (I) nach den Ansprüchen 1 bis 5 mit Streckmitteln und/oder grenzflächenaktiven Mitteln vermischt werden.